# EUROPEAN PATENT APPLICATION

(11) **EP 2 141 177 A1**
(43) Date of publication of application: **06.01.2010**
(21) Application number: 08012149.4
(22) Date of filing: 04.07.2008
(51) Int. Cl.: C07K 14/455, C12N 15/67, A61K 39/015

(54) **MSP-1 protein preparations from Plasmodium**

(71) Applicant: Bujard, Hermann, 69120 Heidelberg (DE)
(72) Inventor: Bujard, Hermann, Prof. Dr., 69120 Heidelberg (DE); Lutz, Rolf, Dr., 69120 Heidelberg (DE); Epp, Christian, Dr., 69120 Heidelberg (DE); Kauth, Christian, Dr., 67655 Kaiserslautern (DE); Wöhlbier, Ute Dr., Richmond, VA 23298-0678 (US)
(74) Representative: Grünecker, Kinkeldey, Stockmair & Schwanhäusser Anwaltssozietät

(57) **Abstract**

The invention relates to a merozoite surface protein 1 ("MSP-1 ") preparation of *Plasmodium falciparum*, said MSP-1 preparation comprising
a) a purified fragment p83/30 of the gp190/MSP-1 from *Plasmodium* without heterologous sequences, and
b) a purified fragment p38/42 of the gp190/MSP-1 from *Plasmodium* without heterologous sequences.

## Description

### Field of the Invention

The invention relates to a merozoite surface protein 1 (MSP-1) from *Plasmodium,* a method of preparing a modified nucleic acid encoding the p83/30 protein subunit thereof, corresponding modified nucleic acids, vectors comprising the same, the corresponding p83/30 subunit of MSP-1, a method of preparing heterodimeric MSP-1, corresponding heterodimeric MSP-1 proteins for use as vaccines, uses of these heterodimeric MSP-1 proteins for making medicaments for preventing and/or treating malaria and a vaccine comprising any of the above nucleic acid, vector or protein products.

### Background of the invention

Malaria is one of the most devastating infectious diseases in the world. According to estimates from the World Health Organisation (WHO) 400 to 900 million incidences of the disease are registered annually. According to information from the Multilateral Initiative against Malaria (MIM) between 700,000 and 2.7 million people die each year from the infection (MIM, 2001). In this respect 40% of the world's population in 99 countries are put at risk from malaria. The disease is caused by single-cell protozoa of the genus *Plasmodium* from the phylum *Apicomplexa*. There are four species which infect humans: *Plasmodium malariae*, responsible for Malaria quartana, *Plasmodium vivax* and *Plasmodium ovale*, both of which cause Malaria tertiana, and finally *Plasmodium falciparum,* the pathogen of Malaria tropica and responsible for almost all fatal infections.

Malaria is again currently spreading to an increasing extent. This is primarily attributable to intensive resistance formation of the malaria pathogen which is promoted in that the medicaments used for the therapy are also recommended and used for prophylaxis. Apart from the search for new chemotherapeutics, research is concentrating on the development of vaccines, because in the course of malaria infections in humans, immunity mechanisms are applied, a fact which is expressed in an increased resistance to the plasmodia, as demonstrated in the development of various types of immunity in humans in regions where malaria epidemics prevail.

### MSP-1 as a potential vaccine

MSP-1 is the main surface protein of merozoites, the invasive form of the blood phases of the malaria pathogen. It is synthesized as a 190-220 kDa precursor which is fixed at the parasite surface via a glycosylphosphatidyl anchor. This protein is processed later during the development of the merozoites into smaller protein fragments which, however, remain non-covalently associated at the merozoite surface up to the invasion of erythrocytes by the parasites.

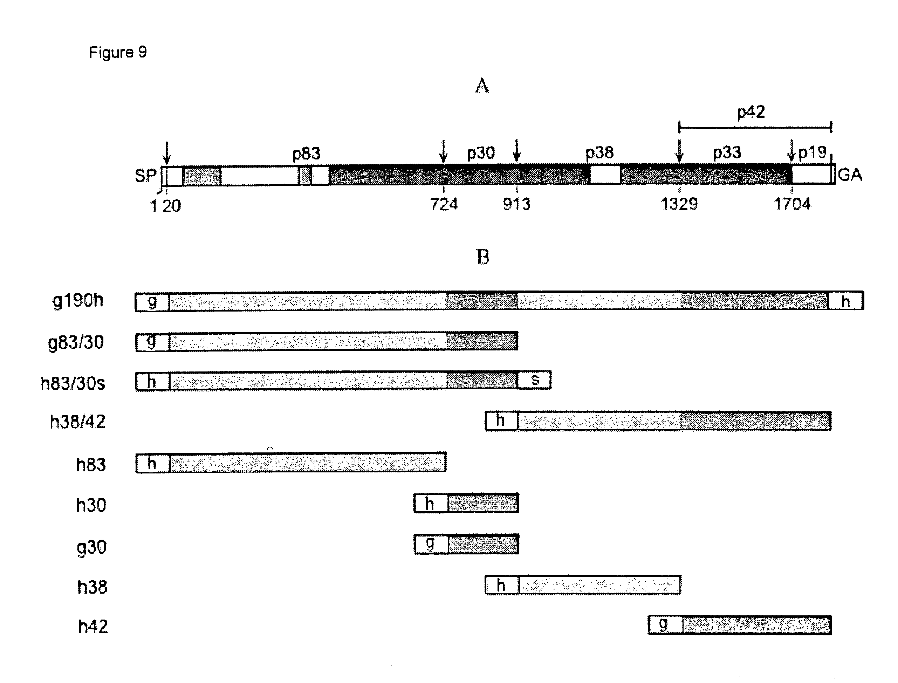

The sequences of the MSP-1 proteins of various *P. falciparum* strains fall into two groups, which have been named after two representative (prototypic) isolates Kᵢ and MAD20. Overall the protein consists of a number of highly preserved regions, dimorphic regions which allow the assignment of a given version of MSP-1 to one of the representative prototypes, and two relatively small oligomorphic blocks in the N-terminal part of the protein (Fig. 9 herein; Tanabe et al. (1987) J. Mol. Biol. 195, 273-87).

The immunization of mice with the protein purified from *P. yoelii* parasites protected the animals from the otherwise fatal infection. Also, the transfer of monoclonal antibodies against MSP-1 from *P. yoelii* provided protection in the mouse model.

Apart from studies on mice, Saimiri and Aotus monkeys have also been immunised with native, immune-affinity purified MSP-1. In these tests the protein obtained from *P. falciparum* partially provided either partial or complete protection against the ensuing infection by the parasite.

Purification of native material from Plasmodia is however expensive and this material cannot be used for production on a large scale. Therefore research into vaccines is concentrated on the development of recombinant vaccines.

For example, mice have been successfully immunized with MSP-1-19 purified from *E. coli* or *Saccharomyces cerevisiae* (Daly and Long (1993) Infect. Immun. 61, 2462-7; Ling et al. (1994) Parasite Immun. 16, 63-7; Tian et al. (1996) J. Immunol. 157, 1176-83; Hirunpetcharat et al (1997) J. Immunol. 15, 3400-11), as well as *Mycobacterium bovis* carrying MSP-1-19 (Matsumoto et al. (1999) Vaccine 18, 832-4). Alternatively to immunization with native or recombinant proteins, DNA coding for MSP-1-19 has also been used as a vaccine and protected immunized mice against infection with *P. chabaudi* (Wunderlich et al. (2000) Infect. Immun. 68, 5839-45).

In Phase I and II studies with MSP-1 fragments as vaccines, their immunogeneity was also shown in humans. In this respect p19 from *P. falciparum* fused onto a T-helper cell epitope of tetanus toxin (Keitel et al. (1999) Vaccine 18, 531-9) and the MSP-1 blocks 3 and 4 (Saul et al. (1999) Vaccine 17, 3145-59; Genton et al. (2000) Vaccine 18, 2504-11) are involved.

Some epidemiological studies in endemic regions show with adults a correlation between antibody titers against MSP-1 and the immunity against malaria (Tolle et al. (1993) Infect. Immun. 61, 40-7; Riley et al. (1992) Parasite Immunol. 14, 321-37; Riley et al. (1993) Parasite Immunol. 15, 513-24). These investigations together with the immunization studies on animals prove that MSP-1 is a promising candidate for the development of a malaria vaccine.

Generally, these studies can be differentiated into two approaches. Either purified material from parasites or material obtained in heterologous systems may be used.

Both for functional investigations and also for use as a vaccine, proteins must be produced reproducibly in a good yield and high quality. MSP-1 can be purified from parasites, but this is only possible on a small scale and with great expense and therefore cannot be carried out for obtaining MSP-1 according to the stated criteria in this way.

Heterologous production of full-length (190 kD) MSP-1 protein is extremely inefficient. Although this full-length protein contains the four major processing fragments p83, p30, p38 and p42 which, after proteolytic processing, form the non-covalently associated heterotetramer, the extremely large size makes efficient heterologous production difficult, severely limiting obtainable yield. Specifically, the high AT-content of 76-78% of the respective parasite genes prevents stable cloning and heterologous expression in good yield. This problem has been solved by synthetic genes encoding the proteins in different codon compositions. Further, recovering a 190 kD protein containing numerous disulfide bonds in its proper conformation, for example from *E. coli,* is difficult experimentally, and wholly unsuited to technical scale preparation required for use of this protein as a vaccine.

Since MSP-1 is not glycosolated in the parasite despite numerous potential glycosolation sites, a prokaryotic expression system would be of advantage for technical scale preparation. However, the folding problems due to multiple disulfide bonds mentioned above persist. Kauth et al. (2003) (Journal of Biological Chemistry 278, 2257-2264) describes the construction of a heteromultimeric MSP-1 complex composed of recombinantly produced MSP-1 protein subunits. Here, synthetic polynucleotides encoding subunits and processing fragments of the MSP-1 complex of *P. falciparum* from strain 3D7 are used to generate each of the four individual MSP-1 subunits individually in highly purified and soluble form from *E. coli*. These subunits are then reassembled to form the MSP-1 heterotetramer. Kauth et al. (2003) describe production of the MSP-1 protein subunits p83, p30, p38 and p42 as well as two synthetic subunit "halves" p83/30 and p38/42. The subunit half p83/30 contains the amino sequences of the p83 and p30 subunits, linked into a single contiguous polypeptide. Likewise, the subunit half p38/42 contains the amino sequences of the p38 and p42 subunits, linked into a single contiguous polypeptide. While Kauth et al. indicates that stable production of MSP-1 protein for the purposes described above may best proceed via production of a heteromultimer, especially a heterodimer of the p83/30 and p38/42 subunits, the heteromultimeric proteins obtained in Kauth et al. (2003) remain ill-suited for the intended application as a vaccine.

First, the MSP-1 subunits of Kauth et al. were labeled with either His₆- or GST-tags so that they could be efficiently purified. These tags were required to isolate a sufficient amount of the subunit in high purity. Such tags are problematic from a regulatory standpoint, and post-expression proteolytic removal of the tags causes great loss in product. Thus, omitting protein tag would comply with regulatory requirements, but would result in a protein which is difficult if not impossible to purify. On the other hand, retaining the protein tag allows purification but precludes any use of the resulting protein as an approved therapeutic/vaccine.

A further problem encountered when constructing a heterodimer of p83/30 and p38/42 in a manner strictly analogous to Kauth et al. is that production of the p83/30 subunit in *E. coli* is very inefficient. Even using an isolation tag, only very low levels of p83/30 are recovered. Moreover, the small amount of the p83/30 fragment produced to begin with is attenuated further still by potent degradation *in vivo*.

There is thus a need for MSP-1 which is efficiently producible, complies with regulatory requirements set for therapeutic proteins, remains isolable in the high yield required for technical preparation and allows the generation of a strong immune response against *P. falciparum.* This need is met by the present invention.

### Brief description of the invention

One aspect of the invention provides a merozoite surface protein 1 ("MSP-1") preparation of *Plasmodium,* said MSP-1 preparation comprising
(a) a purified fragment p83/30 of the gp190/MSP-1 from *Plasmodium* without heterologous sequences, and
(b) a purified fragment p38/42 of the gp190/MSP-1 from *Plasmodium* without heterologous sequences.

A further aspect of the invention provides a composition comprising the above MSP-1 preparation.

The composition of the present invention may be manufactured by a method of preparing a *Plasmodium* MSP-1 containing composition, said method comprising:
a) Expressing the fragment p83/30 of the gp190/MSP-1 protein from *Plasmodium* without heterologous sequences in a host cell;
b) Expressing the fragment p38/42 of the gp190/MSP-1 protein from *Plasmodium* without heterologous sequences in a host cell;
c) Recovering the fragment p83/30 and the fragment p38/42 from the host cells; and
d) Optionally combining the fragments p83/30 and p38/42 in a composition or as a combined preparation.

A further aspect of the invention provides a pharmaceutical composition comprising the above MSP-1 preparation.

A further aspect of the invention provides a method for preparing a modified nucleic acid encoding the p83/30 fragment of MSP-1 of *Plasmodium* having an amino acid sequence as set out in SEQ ID NO: 1, 2 or 3, said method comprising:
a) Providing an mRNA sequence encoding the p83/30 fragment of MSP-1 from *Plasmodium* having an amino acid sequence as set out in SEQ ID NO: 1, 2 or 3;
b) Subjecting the region surrounding the start codon of the mRNA sequence of step (a) to analysis *in silico* to determine the predicted secondary structure of said mRNA sequence;
c) Identifying at least one stable, hairpin-like structure in said region surrounding the start codon of step (b);
d) Modifying the sequence of the mRNA in said region surrounding the start codon of step (b), wherein at least one nucleotide substitution, insertion, deletion and/or inversion destabilizes said hairpin-like region, and wherein the at least one nucleotide substitution, insertion, deletion and/or inversion is effected *in silico* so as to encode the same amino acid sequence as encoded by said unmodified nucleic acid sequence;
e) Subjecting the region of step (d) to analysis *in silico* under the same conditions as set out in step (b);
f) Determining whether the at least one nucleotide substitution, insertion, deletion and/or inversion destabilizes the at least one hairpin-like region containing the at least one ribosomal binding site;
g) Optionally repeating steps (d) - (f) as needed until analysis *in silico* under the same conditions as set out in step (b) indicates the destabilization of the at least one hairpin-like region;
h) Preparing the corresponding modified nucleic acid.

A further aspect of the invention provides a modified nucleic acid encoding the p83/30 subunit of MSP-1 of *Plasmodium* having an amino acid sequence as set out in SEQ ID NO: 1, 2 or 3, wherein the modified nucleic acid is obtainable by the above method.

A further aspect of the invention provides a vector comprising the modified nucleic acid as set out above.

A further aspect of the invention provides a method of preparing the p83/30 fragment of MSP-1 of *Plasmodium,* said method comprising:
a) Providing a modified nucleic acid as set out above and/or a vector as set out above;
b) Incorporating the modified nucleic acid and/or vector of step (a) into a cell, preferably a prokaryotic cell;
c) Incubating the cell of step (b) under conditions allowing expression of the p83/30 subunit of MSP-1; and
d) Recovering the p83/30 fragment of MSP-1.

A further aspect of the invention provides a p83/30 fragment of MSP-1 obtainable by the corresponding preparation method set out above.

A further aspect of the invention provides a method of preparing an MSP-1 preparation from *Plasmodium,* said method comprising:
a) Providing the MSP-1 fragment p38/42 (e.g. having an amino acid sequence as set out in SEQ ID NO: 4 or 5);
b) Mixing said p38/42 fragment of the MSP-1 with the p83/30 subunit of the MSP-1 as set out above (e.g. having an amino acid sequence as set out in SEQ ID NO: 1, 2 or 3); and
c) Recovering the MSP-1 preparation.

A further aspect of the invention provides an MSP-1 preparation as set out above, a modified nucleic acid as set out above, a vector as set out above, an MSP-1 fragment p83/30 as set out above and/or an MSP-1 fragment p38/42 (e.g. having an amino acid sequence as set out in SEQ ID NO: 4 or 5) for use as a vaccine.

A further aspect of the invention provides a use of an MSP-1 preparation as set out above, a modified nucleic acid as set out above, a vector as set out above, an MSP-1 fragment p83/30 as set out above (e.g. as in SEQ ID NO: 1, 2 or 3) and/or an MSP-1 subunit p38/42 (e.g. having an amino acid sequence as set out in SEQ ID NO: 4 or 5) in the manufacture of a medicament for preventing and/or treating malaria.

A further aspect of the invention provides a vaccine comprising an MSP-1 preparation as set out above, a modified nucleic acid as set out above, a vector as set out above, an MSP-1 subunit p83/30 as set out above (e.g. as in SEQ ID NO: 1, 2 or 3) and/or an MSP-1 subunit p38/42 (e.g. having an amino acid sequence as set out in SEQ ID NO: 4 or 5).

### Description of the figures

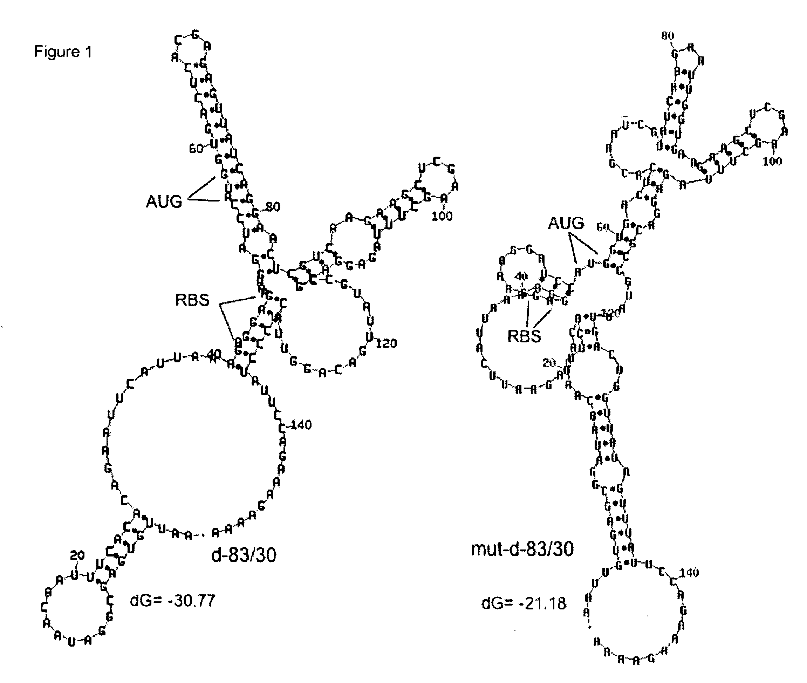

- Fig. 1: Secondary mRNA structures of MSP-1 p83/30 before (left) and after (right) sequence modifications, as predicted *in silico.* The comparison illustrates the marked modification of predicted secondary mRNA structure achieved by destabilizing mRNA secondary structure known or suspected of harboring ribosomal binding sites. The effect of the destabilization was analyzed by *in silico* substitution of single nucleotides. The first 150 bp of the p83/30 mRNA were subjected to *in silico* analysis of secondary structure by using the "mfold" program by Zuker and Turner (version 3.2) provided by the web server of Rensselaer Polytechnic Institute, Troy, NY, USA (for references see hereinbelow). Nucleotides of ribosomal binding site ("RBS") and base-pairing sequences are as indicated. The translation start site (AUG) is also indicated.

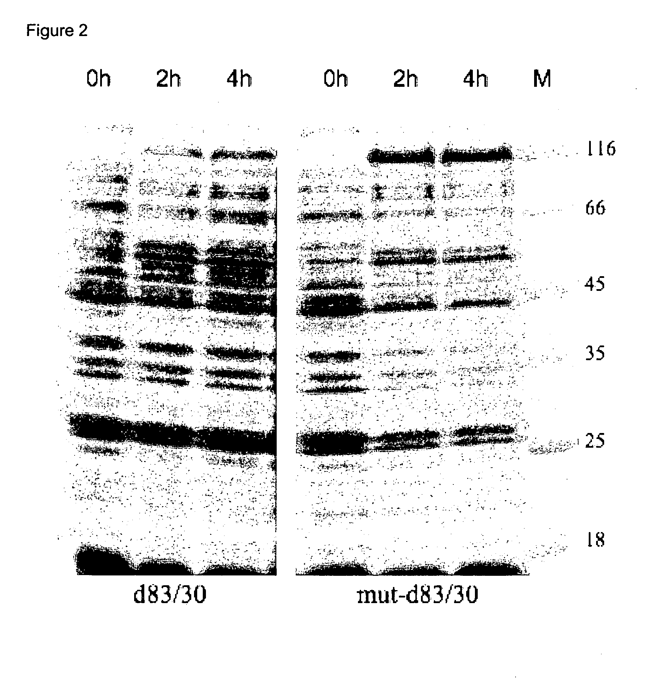

- Fig. 2:: Comparison of the amount of crude protein expression product obtained using unmodified mRNA encoding MSP-1 fragment p83/30 (left, from *Plasmodium falciparum* strain 3D7) and mRNA in which regions of secondary hairpin-like structure containing ribosomal binding sites and/or translation initiation sites have been mutated to destabilize the hairpin-like structure (right) in *E. coli.* MSP-1 protein subunit p83/30 is represented by the dark band in the 2 hour and 4 hour lanes of the gels for both the native and mutated sequences at approximately 116 kD, as measured by the marker ladder at far right. As can be seen by comparing the respective 2 hour and 4 hour lanes in the left and right panels, modification of the MSP-1 p83/30 fragment led to a higher amount of expressed protein (right) than the corresponding unmodified sequence (left).

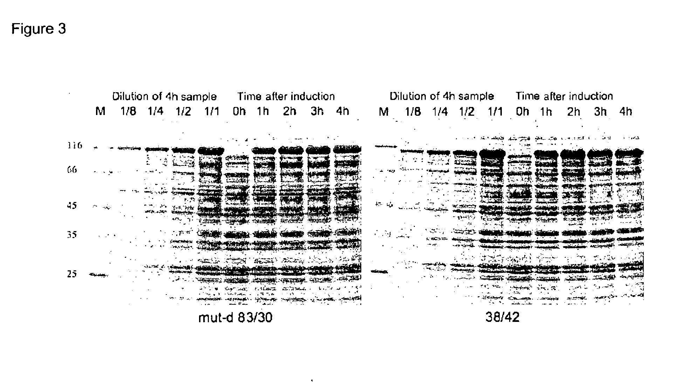

- Fig. 3:: Time course of the production of MSP-1 halves p83/30 (left, based on mutated nucleic acid sequence corresponding to SEQ ID NO: 8 or 9) and p38/42 (right, based on a nucleic acid sequence corresponding to SEQ ID NO: 10). Lanes 1 and 11 (counted from left) show molecular weight marker ladders. Lanes 2-5 and 12-15 (counted from left) show a dilution series for p83/30 and p38/42 fragments of MSP-1, respectively, based on the highest amount of protein obtained after 4 hours of expression. Lanes 6-10 and 16-20 show a time course over 0, 1, 2, 3 and 4 hours of expression for the respective MSP-1 halves p83/30 and p38/42. In each case, the accumulation of protein expression product corresponding approximately to 116 kD corresponds to the desired respective MSP-1 half. 2-fold dilutions up to 1/8 of the original concentration were prepared from the 4h sample. Samples were analyzed by SDS-PAGE and Coomassie staining.

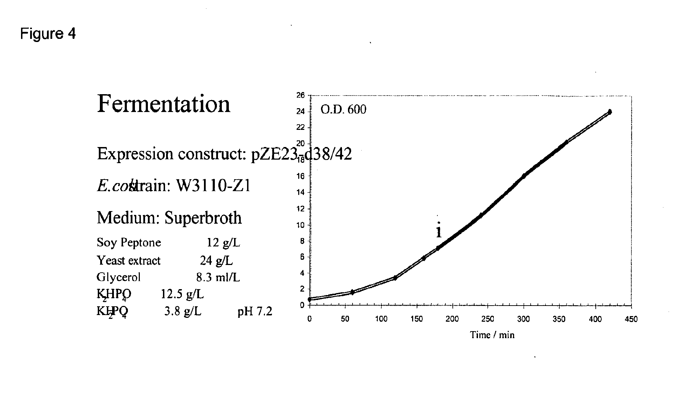

- Fig. 4:: A typical fermentation process in a 15 liter fermenter (Braun, Biostat) shown by example of W3110Z2-p38/42. A typical fermentation process in a 15 liter fermenter is shown. The growth of the culture with time is shown. Induction of expression of the p38/42 fragment of MSP-1 took place at the timepoint indicated by the letter "i" in the figure.

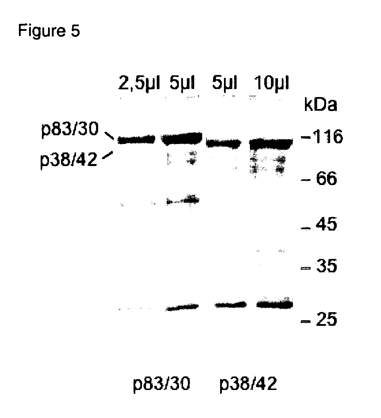

- Fig. 5: Electrophoretic analysis of solubilized IBs. The positions of the two MSP-1 fragments p83/30 and p38/42 are indicated at the side of the gel. SDS-PAGE was performed with 10% polyacrylamide under reducing conditions, followed by Coomassie staining.

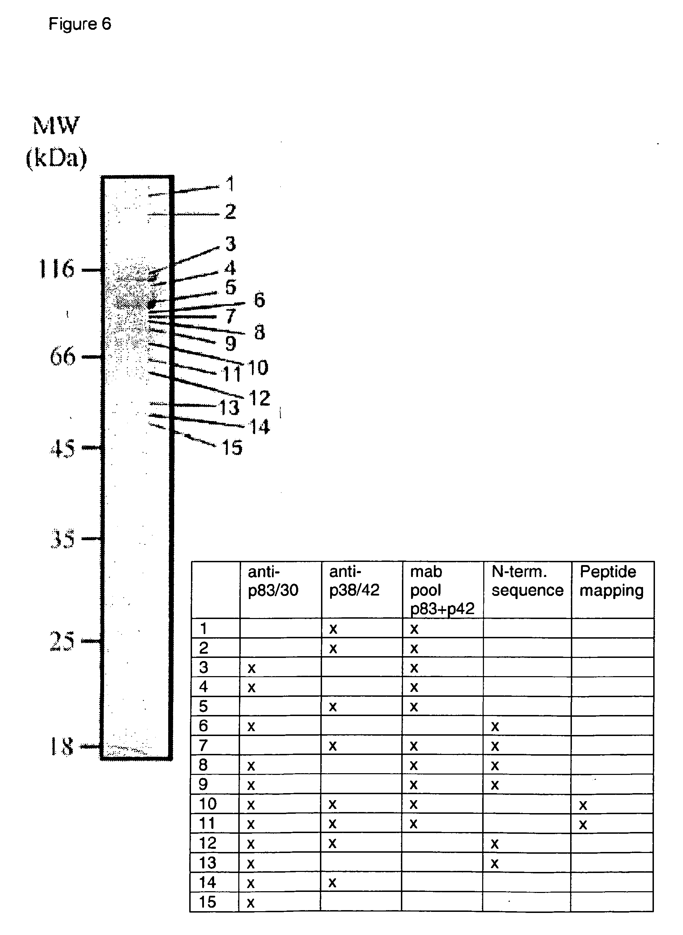

- Fig. 6: Summary of the characterization of proteins present in MSP-1 preparations. The 15 most abundant protein bands are numbered, and were identified by at least one of the following methods (indicated in the title row of the table to the right of the figure): Western Blot with rabbit sera specific for p83/30 ("anti-p83/30), Western Blot with rabbit sera specific for p38/42 ("anti-p38/42), pool of monoclonal antibodies specific for p83 or p42 ("mab pool p83+p42"), direct identification by N-terminal sequencing ("N-term. sequence") and peptide mapping using mass spectrometry ("peptide mapping"). The numbers for the gel bands correspond to the numbers in the leftmost column in the table, with an "x" in the table indicating that a certain analysis method was applied to the protein in the correspondingly numbered band. As can be seen, all detectable components could be identified either by N-terminal sequencing, by peptide mapping via mass spectroscopy or by appropriate monoclonal/polyclonal antibodies against the p83/30 and/or p38/42 fragments of MSP-1.

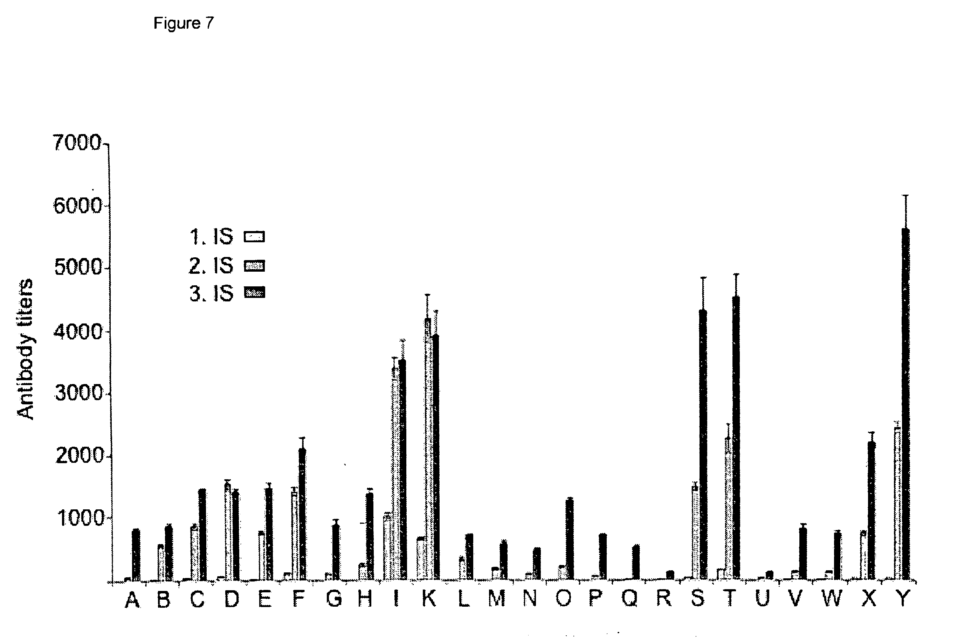

- Fig. 7:: Antibody titers in mouse sera obtained after immunization with MSP-1 in different formulations. Higher bars indicate higher antibody titres, indicating a stronger (and therefore more desirable) immune response to a respective protein. Titers were determined by ELISA using MSP-1 heterodimeric protein made of p83/30 and p38/42 as capture antigen. A: PBS; B: aluminum hydroxide; E: aluminum phosphate; H: MPL (Sigma); I, CpG (Qiagen); L - Q: IC31; R: PBS; S: Montanide ISA720; T: Montanide ISA51: U - W: Virosomes; X, Y: FCA/FIA. IS.1 - IS.3: Sera from first, second or third immunisation.

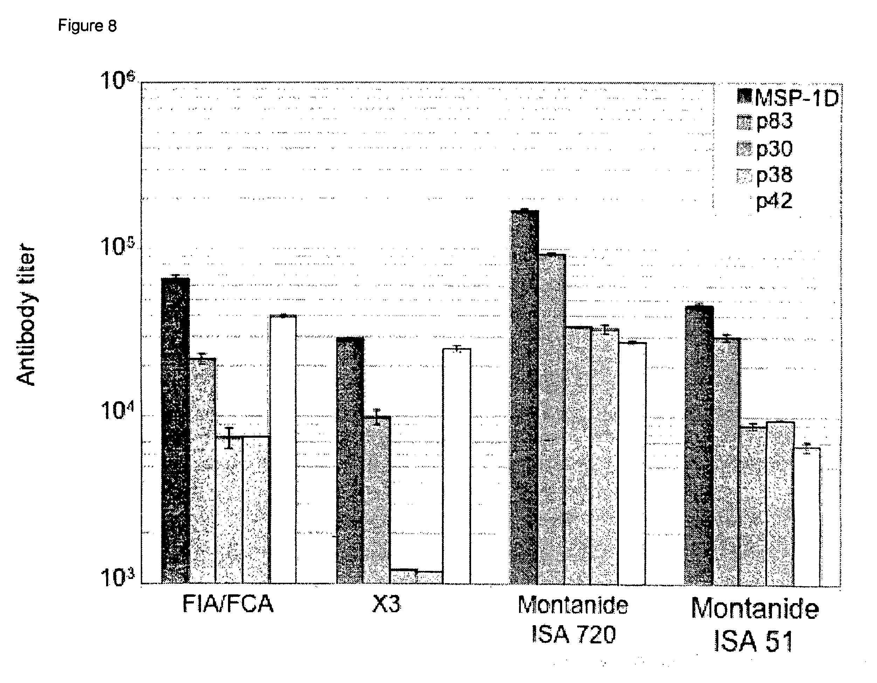

- Fig. 8:: Rabbit antibody titers following immunization with heterodimeric MSP-1 produced as indicated herein (indicated as "MSP-1 D" at the upper right of the figure), p83, p30, p38 and p42 together with various adjuvants. Higher bars indicate higher antibody titers, indicating a stronger (and therefore more desirable) immune response to a respective protein. The bars represent results from ELISA studies. Equal amounts of a respective protein as antigen were administered to rabbits (100 µg per injection). The adjuvants used together with the various proteins are indicated below a respective cluster of result bars along the x-axis. The adjuvant "X1" denotes an adjuvant obtained under a secrecy agreement. As can be seen in the third cluster of result bars, administration of MSP-1 heteromultimer, as well as selected fragments thereof together with montanide, ISA720 led to significantly higher antibody titres in rabbit than observed when same proteins were administered with other adjuvants. This is especially the case for the MSP-1 heterodimer as prepared herein as well as for the individual MSP-1 protein subunits p83, p30 and p38.

- Fig. 9:: Schematic depiction of the primary structure of the MSP-1 derived from *P. falciparum* strain 3D7 as "MS-1 D", a representative of the MAD20 prototype and various fragments thereof which have been recombinantly produced. The numbers below the scheme denote amino acid positions. "SP" denotes "signal peptide. "GA" denotes a glycosynphosphatidylinositol moiety. The arrows at the top of Fig. 9A indicate the sites where the precursor protein is normally cleaved into its major subunits p83 to p42, which are shown in Fig. 9B. A secondary proteolysis cleaves p42 into p33 and p19. The amino acid positions delineate heterologously produced fragments p83, p30, p38 and p42. The isolation tag designations "g", "h" and "s" refer to GST, His₆ and Strep tags, respectively. MSP-1 fragments p83/30 and p38/42, are shown in rows 2 and 4, respectively, of Fig. 9B. In this figure, these fragments bear heterologous isolation tags which can be advantageously omitted under the present invention.

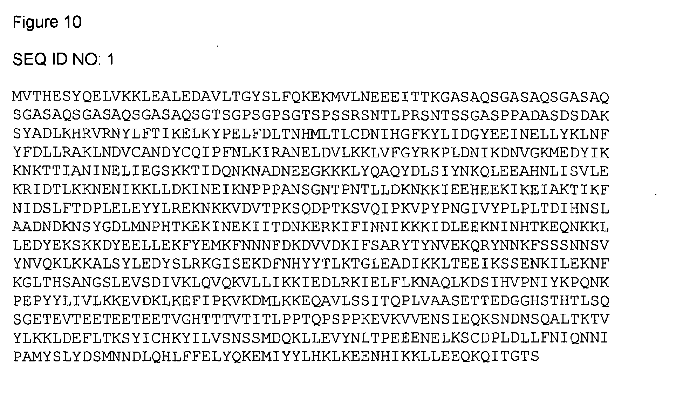

- Fig. 10:: SEQ ID NO: 1. Amino acid sequence of p83/30 from *Plasmodium falciparum* strain 3D7.

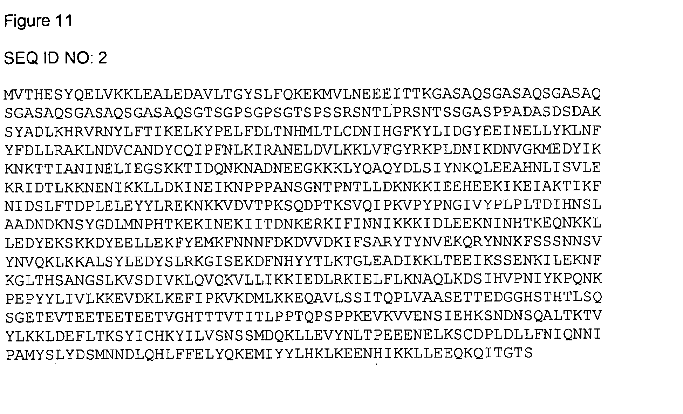

- Fig. 11:: SEQ ID NO: 2. Amino acid sequence of p83/30 from *Plasmodium falciparum* strain 3D7, with two amino acid mutations: E612K and Q767H.

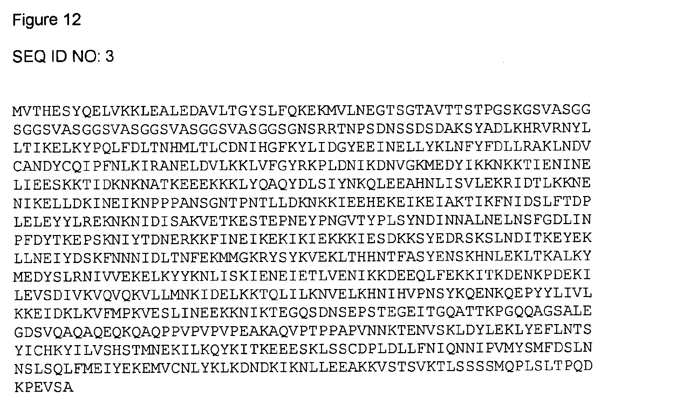

- Fig. 12:: SEQ ID NO: 3. Amino acid sequence of p83/30 from *Plasmodium falciparum* strain FCB-1.

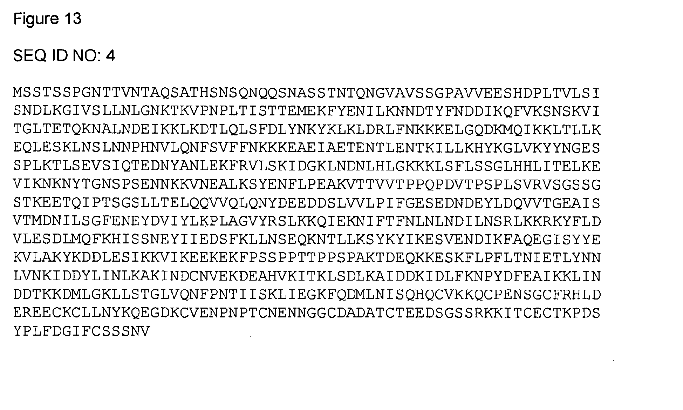

- Fig. 13:: SEQ ID NO: 4. Amino acid sequence of p38/42 from *Plasmodium falciparum* strain 3D7.

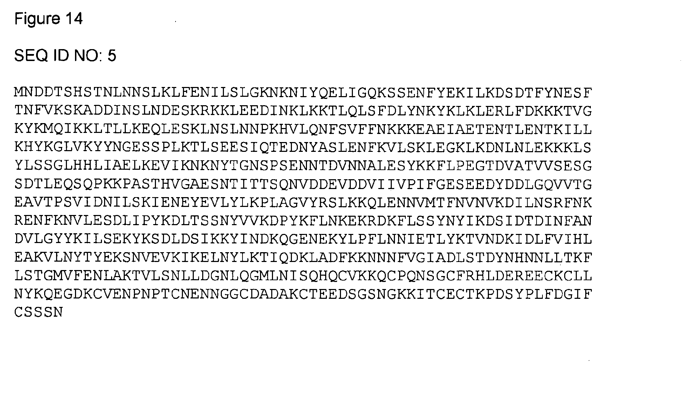

- Fig.14:: SEQ ID NO: 5. Amino acid sequence of p38/42 from *Plasmodium falciparum* strain FCB-1.

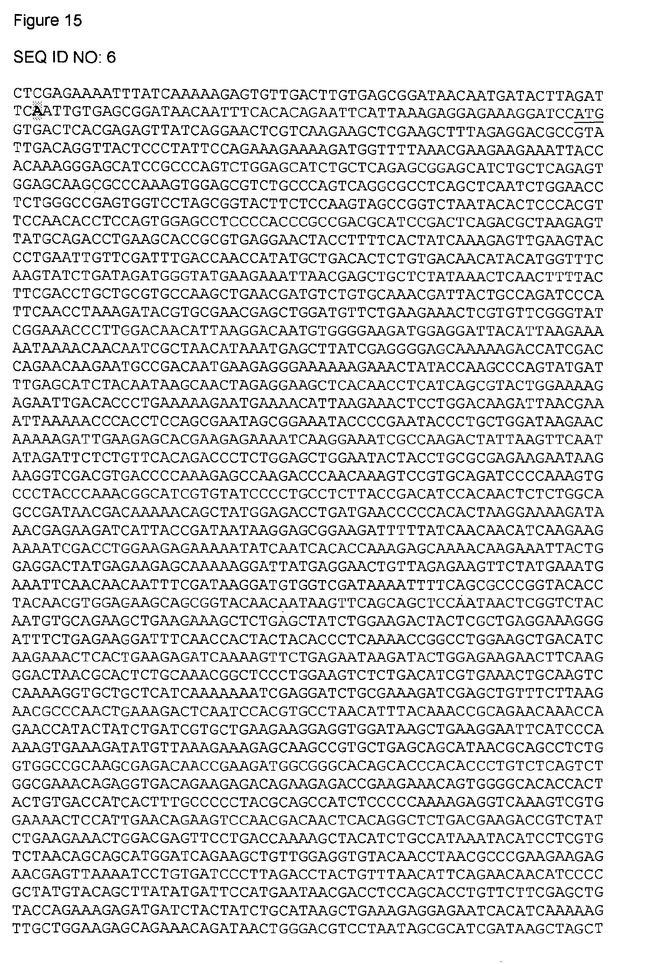

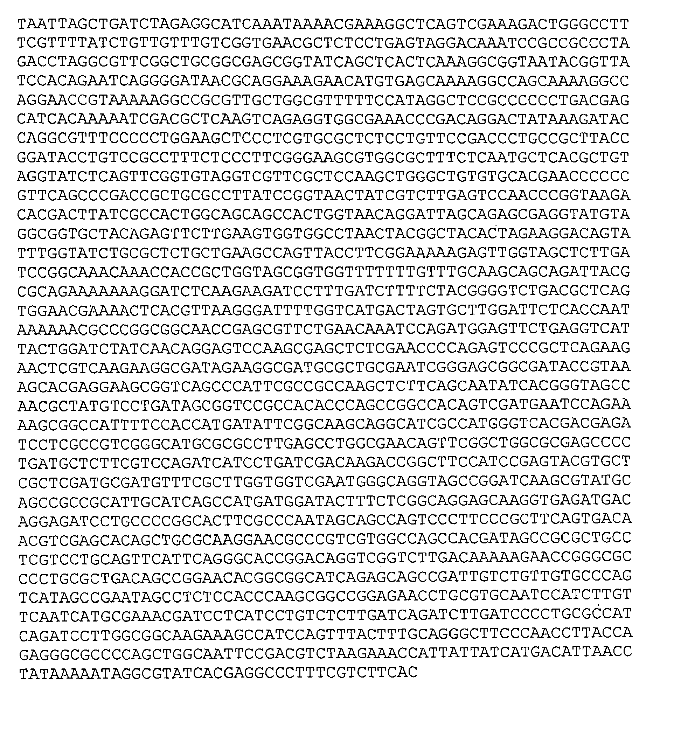

- Fig. 15:: SEQ ID NO: 6. Nucleotide sequence of pZE23/d-83/30 (plasmid encoding p83/30 from *Plasmodium falciparum* strain 3D7 corresponding to SEQ ID NO: 1, i.e. without silent mutations in the RBS and without amino acid mutations E612K and Q767H). The transcriptional start nucleotide A63 is indicated in **shadowed boldface** and the start codon ATG is underlined.

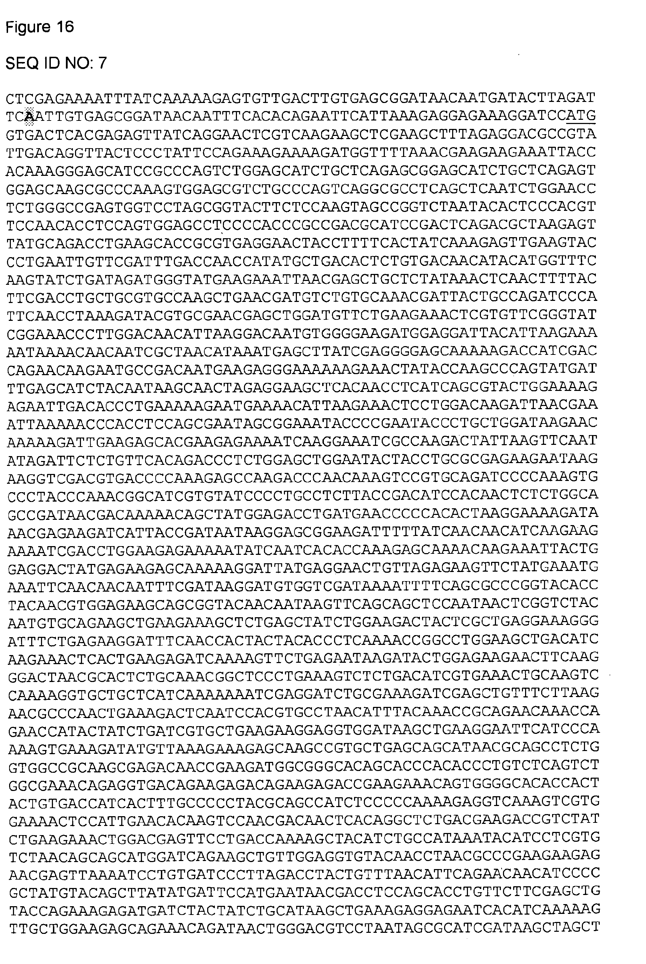

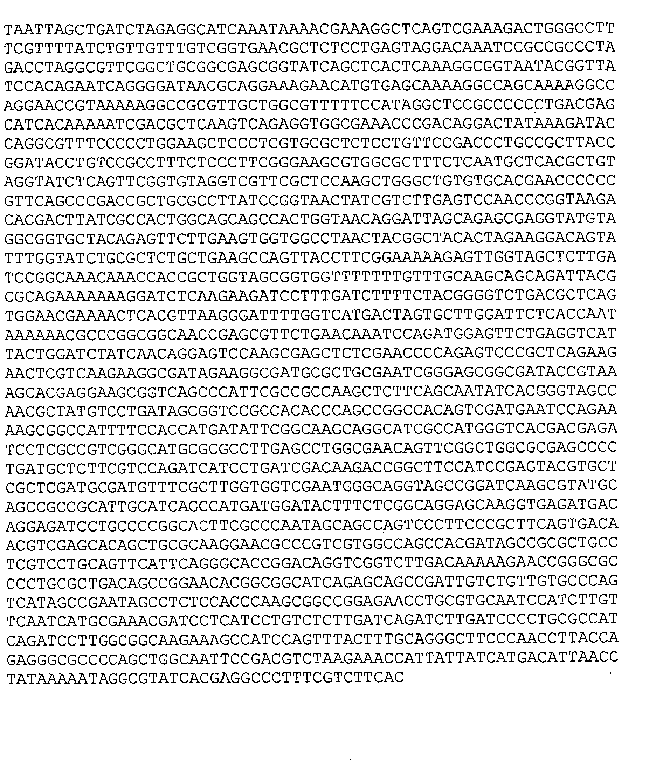

- Fig. 16:: SEQ ID NO: 7. Nucleotide sequence of pZE/d-p83/30 (plasmid encoding p83/30 from *Plasmodium falciparum* strain 3D7 corresponding to SEQ ID NO: 2, i.e. without silent mutations in the RBS but with amino acid mutations E612K and Q767H). The transcriptional start nucleotide A63 is indicated in **shadowed boldface** and the start codon ATG is underlined.

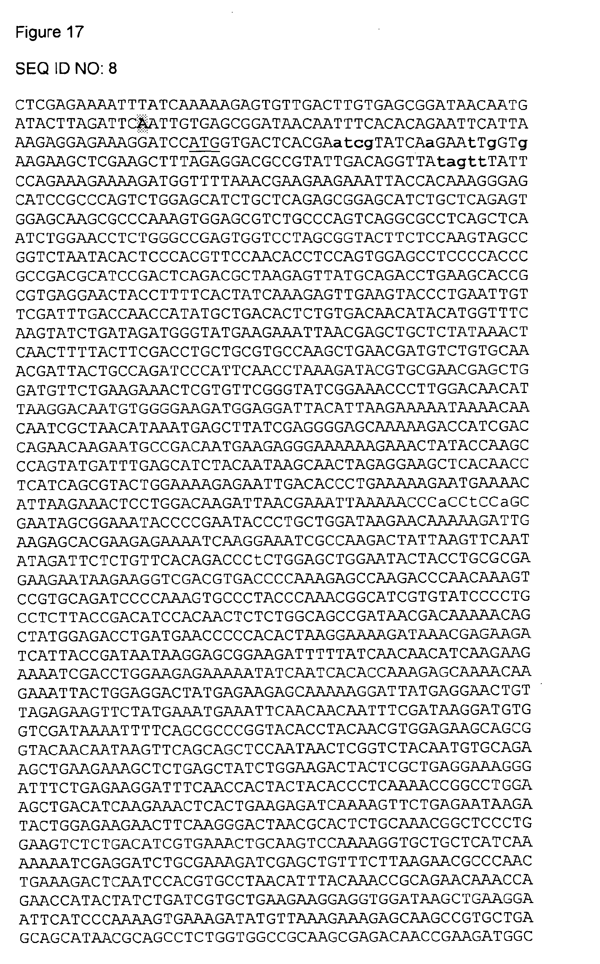

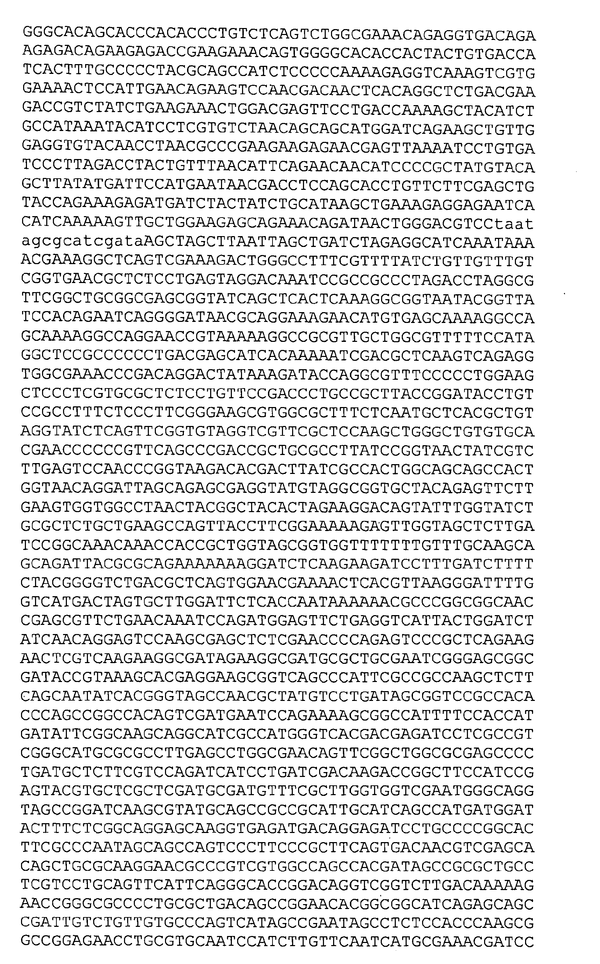

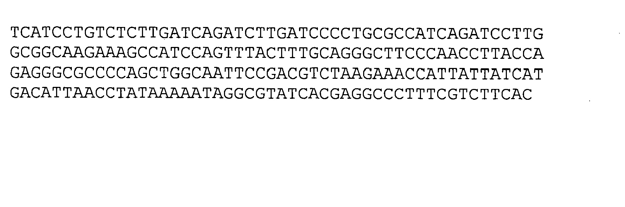

- Fig. 17:: SEQ ID NO: 8. Nucleotide sequence of pZE23/d-p83/30 (plasmid encoding p83/30 from *Plasmodium falciparum* strain 3D7 corresponding to SEQ ID NO: 1, i.e. with silent mutations in the RBS but without amino acid mutations E612K and Q767H). The transcriptional start nucleotide A63 is indicated in **shadowed boldface,** the start codon ATG is underlined, and the nucleotide modifications in the region of the ribosomal binding site are in **lower case boldface**.

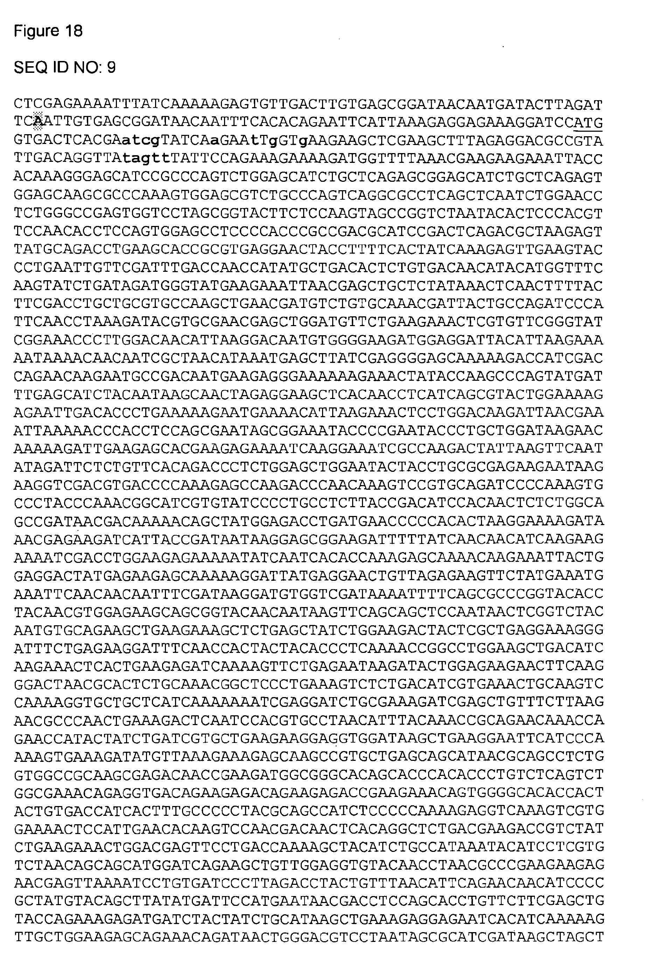

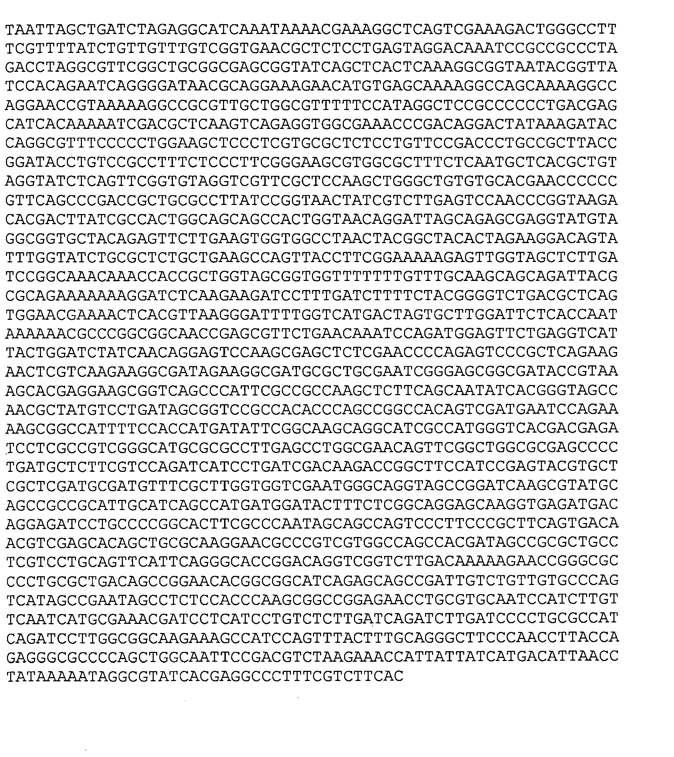

- Fig. 18:: SEQ ID NO: 9. Nucleotide sequence of pZE23/d-83/30 (plasmid encoding p83/30 from *Plasmodium falciparum* strain 3D7 corresponding to SEQ ID NO: 2, i.e. with silent mutations in the RBS and with amino acid mutations E612K and Q767H). The transcriptional start nucleotide A63 is indicated in **shadowed boldface,** the art codon ATG is underlined, and the nucleotide modifications in the region of the ribosomal binding site are in **lower case boldface.**

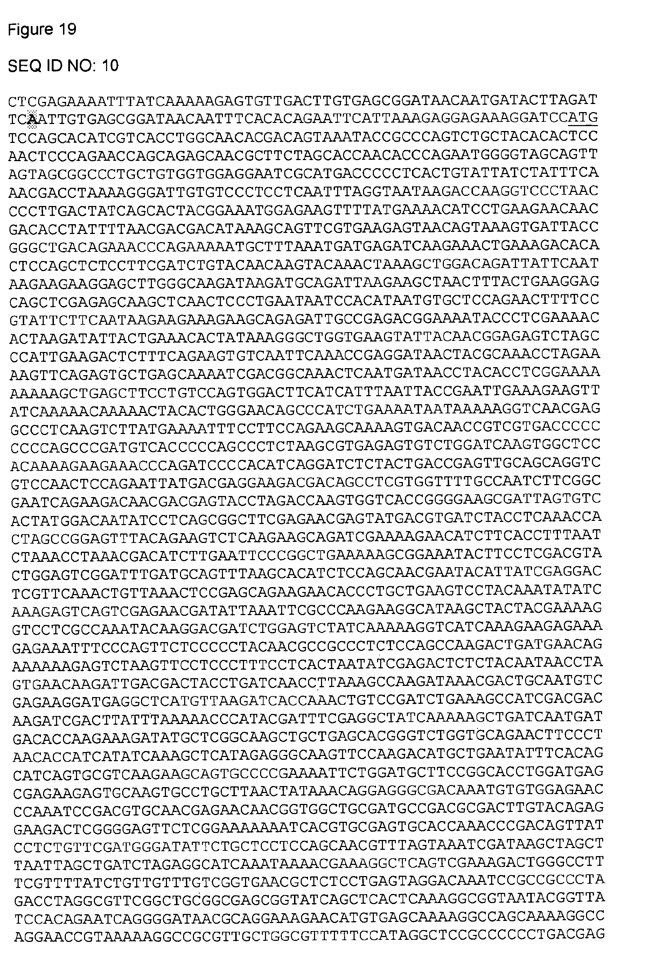

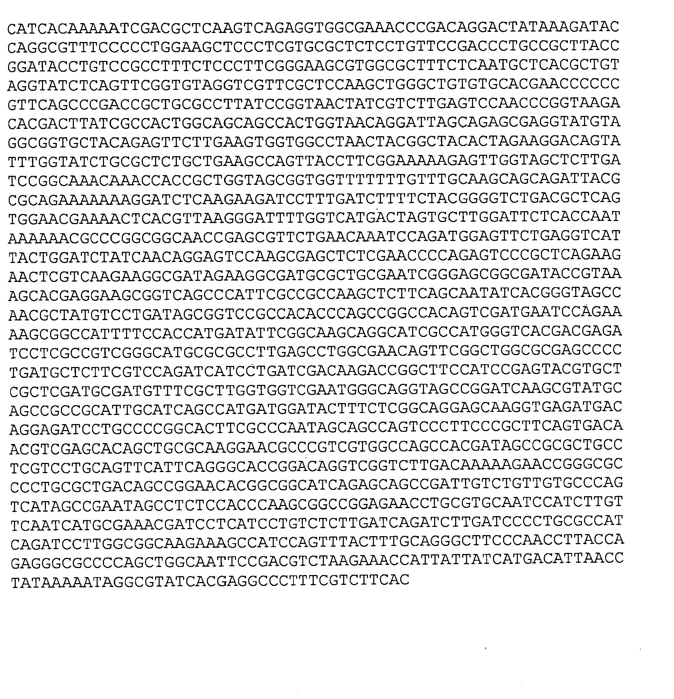

- Fig. 19:: SEQ ID NO: 10. Nucleotide sequence of pZE23/d-38/42 (plasmid encoding p38/42 from *Plasmodium falciparum* strain 3D7 corresponding to SEQ ID NO: 4). The transcriptional start nucleotide A63 is indicated in **shadowed boldface** and the start codon ATG is underlined.

### Detailed description of the invention

As indicated above, one aspect of the invention provides a merozoite surface protein 1 ("MSP-1") preparation from *Plasmodium,* said MSP-1 preparation comprising
(a) a purified fragment p83/30 of the gp190/MSP-1 from *Plasmodium* without heterologous sequences, and
(b) a purified fragment p38/42 of the gp190/MSP-1 from *Plasmodium* without heterologous sequences.

The MSP-1 preparation according to this aspect of the invention is advantageous in that it elicits a strong immune response in host animals, rendering it suitable for use as the active agent in a malaria vaccine. At the same time, the protein is compliant with the Good Manufacturing Practice ("GMP") requirement that therapeutic proteins intended for administration may not contain isolation tags, for example His₆, as such tags interact with/chelate substances in the body of the patient. It should be emphasized that without isolation tags, the MSP-1 would normally have proven difficult to recover at all, let alone in the amounts required for preparation of a vaccine based on this protein. As described hereinbelow, the present inventors have determined how to isolate and recover sufficient amounts of MSP-1 protein as a heterodimer preparation for technical scale preparation of medicaments while complying with regulatory requirements set for therapeutic protein intended for administration to patients. As such, the MSP-1 preparation of the present invention satisfies an unmet need.

As used herein in this and other aspects/embodiments, the term "MSP-1 preparation" includes MSP-1 comprising two associated, preferably two non-covalently associated protein subunits or fragments ("subunit" and "fragment" being used interchangeably throughout the present application), wherein the two protein subunits are not the same. The term "MSP-1 preparation" thus encompasses heterodimeric MSP-1. For example, the MSP-1 subunits p83/30 and p38/42 are different polypeptides which spontaneously associate non-covalently with one another to form an MSP-1 heterodimer with immunological properties, i.e. epitopes, mirroring those of the native MSP-1 heterotetramer. The term "MSP-1" may also include incomplete portions of a full MSP-1 protein, for example p83/30 or p38/42 "halves" of heterodimeric MSP-1 as well as fragments thereof. In these cases, for purposes of clarity, the respective "half" will generally be referred to as an "MSP-1 fragment" or an "MSP-1 subunit". For purposes of readability, the MSP-1 preparation will be referred to hereinafter simply as "MSP-1", but it is understood that "MSP-1 preparation" as defined above is meant.

As used herein in this and other aspects/embodiments, the term "heterologous sequences" refers to sequences which are not MSP-1 sequences. For example, a part of a polypeptide, for example an extension of a desired polypeptide at the N- and/or C-terminal end, comprising a region of specific sequence intended to specifically interact with a predetermined binding partner may be considered a "heterologous sequence". Such "isolation tags" are commonly applied in protein chemistry to aid in the isolation/recovery of recombinantly produced proteins. Examples of commonly used heterologous tags include His₆ (which chelates divalent nickel), glutathione S-transferase ("GST", which binds glutathione), and "strep" (8-amino acid tag which binds to engineered streptavidin). As used herein in this and other aspects/embodiments, the term "heterologous sequence" also refers to any chemical modification of an expressed protein which is effected with the purpose of enabling protein recovery by selective binding methods. MSP-1 of the present invention comprises no such heterologous sequences.

As used herein in this and other aspects/embodiments, the term "purified fragment" (referring to p83/30) refers to a single polypeptide comprising the p83 portion and the p30 portion of MSP-1 of *Plasmodium.* Preferably, MSP-1 protein comprises a p83/30 fragment from a *Plasmodium* F-strain or a *Plasmodium* D-strain. The purified p38/42 fragment is a single polypeptide comprising the p38 portion and the p42 fragment of MSP-1 from *Plasmodium.* The p38/42 fragment may be derived from any *Plasmodium* strain, e.g. a D-strain or an F-strain of *Plasmodium falciparum.*

As used herein in this and other aspects/embodiments, the terms "p83/30 fragment" and "p38/42 fragment" also refer to modified fragments which have an amino acid sequence identity of at least 90%, preferably of at least 95% and more preferably of at least 98% over the entire length of the polypeptide to a native p83/30 or p38/42 fragment. The terms also encompass truncated fragments which may comprise deletions of single amino acids and/or amino acid portions of up to 10, more preferably up to 5 amino acids compared to the wild-type polypeptide. A p83/30 fragment preferably has a length of at least 800 amino acids, more preferably of at least 850 amino acids. Further, the p83/30 fragment has a sequence identity of at least 90%, more preferably at least 95% and, most preferably, at least 98% with the p83/30 fragment of the *P. falciparum* F-variant in SEQ ID NO: 3 or the *P. falciparum* D-variants of either SEQ ID NO: 1 or 2.

The MSP-1 preparation of the present invention as well as compositions comprising the inventive MSP-1 preparation advantageously do not comprise any fragments or other polypeptide products not deriving from the MSP-1 fragments p83, p30, p38 or p42.

According to one embodiment, the heterologous sequence is an isolation tag.

According to a further embodiment, the purified p83/30 fragment is an F-fragment, i.e. a fragment derived from an F-strain of *Plasmodium,* particularly from *Plasmodium falciparum* strain FCB-1 also known as FC or F. It has been found that the F-fragment is more stable against proteolytic degradation than the D-fragment, i.e. a fragment derived from a D-strain of *Plasmodium,* particularly from the *Plasmodium falciparum* strain 3D7 known as NF54. It has also been found that the p83/30 fragment may be combined with a heterologous or a homologous p38/42 fragment, for example with a heterologous p38/42 D-fragment and/or a p38/42 F-fragment.

Components a) and b) are preferably recombinant polypeptides, i.e. polypeptides which have been manufactured in a recombinant host cell, e.g. a eukaryotic host cell such as a yeast cell, e.g. *S. cerevisiae* or *P. pastoris*, or in prokaryotic cells, e.g. gram-negative bacterial cells such as E. coli. Preferably, the recombinant host cell is an E. coli cell. More preferably, the host cell is *E. coli* W3110Z2, for example E. coli W3110Z2-pZE23/d-83/30 (DSM accession number 16602) or W3110Z2-pZE23/d-38/42 (DSM accession number 16600). The DSM numbers represent those assigned by the ***D**eutsche **S**ammlung von **M**ikroorganismen und Zellkulturen* (German Collection of Microorganisms and Cell Cultures), in Braunschweig, Germany.

In one preferred embodiment, the p83/30 fragment preferably comprises the amino sequence of an F-strain of *P. falciparum* as shown in SEQ ID NO: 3 and optionally an N-terminal signal peptide sequence or a modified F-fragment derived from the F-strain sequence. Alternatively, the p83/30 fragment comprises the amino acid sequence of a D-strain of *P. falciparum* and optionally an N-terminal signal peptide sequence of a modified D-strain sequence. The amino acid sequence shown in SEQ ID NO: 2 is derived from the amino acid sequence of a D-strain and comprises 2 amino acid substitutions at positions 612 (E → K) and 767 (Q → H). As a further alternative, the p83/30 fragment comprises the amino acid sequence of a D-strain of *P. falciparum* as shown in SEQ ID NO: 1 and optionally an N-terminal signal peptide sequence of a modified D-strain sequence.

The p38/42 fragment may be derived from an F-strain of *P. falciparum* as shown e.g. in SEQ ID NO: 5 and/or from a D-strain of *P. falciparum* as shown in SEQ ID NO: 4. It was surprisingly found that a p83/30 fragment of an F-strain may be combined with both a homologous p38/42 fragment from an F-strain or with a heterologous p38/42 fragment from a different *P. falciparum* strain, e.g. a D-strain.

The p38/42 fragment preferably has a length of at least 700 amino acids and more preferably at least 750 amino acids. Further, it is preferred that the p38/42 fragment has a sequence identity of at least 90%, more preferably at least 95% and, most preferably, at least 98%, compared to the p38/42 fragments from a *P. falciparum* F-strain (SEQ ID NO: 5) and/or from a D-strain (SEQ ID NO: 4).

The amino acid sequences of particularly preferred embodiments of p83/30 and p38/42 are shown in SEQ ID NO: 1-5, and preferred combinations of the p83/30 fragment of MSP-1 with the p38/42 fragment of MSP-1 to form heterodimeric MSP-1 are thus as set out in the following Table 1:

**Table 1**

| **p83/30 fragment** | **p38/42 fragment** |
|---|---|
| SEQ ID NO: 1 | SEQ ID NO: 4 |
| SEQ ID NO: 1 | SEQ ID NO: 5 |
| SEQ ID NO: 2 | SEQ ID NO: 4 |
| SEQ ID NO: 2 | SEQ ID NO: 5 |
| SEQ ID NO: 3 | SEQ ID NO: 4 |
| SEQ ID NO: 3 | SEQ ID NO: 5 |

In this and other aspects/embodiments of the present invention, it is preferred that the *Plasmodium* species is *Plasmodium falciparum.*

In a further aspect the present invention provides a method for preparing a modified nucleic acid encoding the p83/30 fragment of MSP-1 from *Plasmodium* having an amino acid sequence as set out in SEQ ID NO: 1, 2 or 3, said method comprising:
a) Providing an mRNA sequence encoding the p83/30 subunit of MSP-1 from *Plasmodium* having an amino acid sequence as set out in SEQ ID NO: 1, 2 or 3;
b) Subjecting the region surrounding the start codon of the mRNA sequence of step (a) to analysis *in silico* to determine the predicted secondary structure of said mRNA sequence;
c) Identifying at least one stable, hairpin-like structure in said region surrounding the start codon of step (b);
d) Modifying the sequence of the mRNA in said region surrounding the start codon of step (b), wherein at least one nucleotide substitution, insertion, deletion and/or inversion destabilizes said hairpin-like region, and wherein the at least one nucleotide substitution, insertion, deletion and/or inversion is effected *in silico* so as to encode the same amino acid sequence as encoded by said unmodified nucleic acid sequence;
e) Subjecting the region of step (d) to analysis *in silico* under the same conditions as set out in step (b);
f) Determining whether the at least one nucleotide substitution, insertion, deletion and/or inversion destabilizes the at least one hairpin-like region containing the at least one ribosomal binding site;
g) Optionally repeating steps (d) - (f) as needed until analysis *in silico* under the same conditions as set out in step (b) indicates the destabilization of the at least one hairpin-like region;
h) Preparing the corresponding modified nucleic acid.

Previously, preparation of sufficient quantities of heterodimeric MSP-1 comprising the MSP-1 protein subunits p83/30 and p38/42 has been limited by the low expression of the p83/30 subunit. The present inventors have effectively removed this bottleneck in the preparation of MSP-1 comprising the p83/30 and p38/42 subunits by determining and eliminating the source of the low expression observed. Specifically, the inventors have determined that the region surrounding the start codon in the unmodified mRNA encoding the p83/30 subunit of MSP-1 (e.g. with amino acid sequence as set out in SEQ ID NO: 1, 2 or 3) contains multiple regions of secondary structure, for example hairpin-like regions, which impede recognition by the ribosomal machinery required for translation. By determining which regions of the mRNA exist in stable secondary structure, for example hairpin-like structures, the inventors have determined that targeted modifications to these regions of the nucleic acid sequence can be effected so as to destabilize the secondary structure and render previously inaccessible regions of mRNA available for recognition by ribosomes. Since protein expression levels depend in great part on proper ribosomal recognition of its binding sites on mRNA, rendering these sites more accessible to the ribosome leads to greatly increased expression levels of the corresponding protein, thus eliminating the previously limiting step in the recombinant production of MSP-1.

As used herein in this and other aspects/embodiments, the term "modified" refers to the fact that at least one change has been made in the coding nucleotide sequence of a starting nucleic acid, said change resulting in the destabilization of the determined or suspected regions of secondary hairpin-like structure in the corresponding mRNA of this nucleic acid. However, a modified nucleic acid need not be mRNA as such. While the term "modified nucleic acid" includes mRNA, it also encompasses a DNA sequence which, when transcribed into mRNA, will have no or at least reduced or different secondary hairpin-like structure in the regions corresponding to ribosomal binding sites, as compared to the corresponding nucleic acid sequence prior to modification. Further, when the modification effected is within the ORF encoding the p83/30 MSP-1 protein subunit, the modification preferably results in a nucleotide triplet which, due to the degeneracy of the genetic code, is translated as the same amino acid as would result from translation of the corresponding triplet in unmodified form. This is referred to in this and other aspects/embodiments as a "silent modification" or a "silent mutation".

As used herein in this and other aspects/embodiments, the term "region surrounding the start codon" refers to a window of nucleotides starting at the transcriptional start site, and extending over the AUG start codon about 100-150 nucleotides into the coding region, to comprise a total window length of about 150-200 nucleotides.

As used herein in the present and other aspects, the term "about" typically denotes variance of up to and including ±15% (explicitly including all intermediate values of whole or decimal value between 0-15%), the result of this variance being rounded up or down to the nearest whole number as appropriate.

As used herein in this and other aspects/embodiments, the term "ribosomal binding site" refers to stretches of nucleotides known to be bound by ribosomes as part of the process of translating mRNA into an amino acid sequence. The skilled person understands how to recognize these sequences. For example, in prokaryotes the ribosomal binding site, also referred to as the "Shine-Dalgarno sequence" or the "Shine-Dalgarno box" (after Shine & Dalgarno (1975) Nature 254, 34-8) consists of purine-rich sequences approximately 10 nucleotides upstream of the start codon. The ribosomal binding site sequence used for the expression of the p83/30 fragment of MSP-1 under the present invention corresponds to the Shine-Dalgamo consensus sequence, the ability of which to drive gene expression in E. coli has been previously described (Lutz & Bujard (1997) Nucleic Acids Res. 25, 1203-10

As used herein in this and other aspects/embodiments, the term "*in silico*" refers to analysis done electronically, for example on a computer using appropriate software for the analysis of nucleic acids and their predicted secondary structure. Suitable software platforms for nucleic acid sequence analysis are known to the skilled person, for example the "mfold" program by Zuker and Turner (version 3.2) provided by the web server of Rensselaer Polytechnic Institute, Troy, NY, USA (Zuker (2003) Nucleic Acids Res. 31 (13), 3406-15; Mathews et al. (1999) J. Mol. Biol. 288, 911-40). Alternative sources are the "Vienna RNA package" (available on the internet under: http://www.tbi.univie.ac.at/∼ivo/RNA/) or "RNA world" at IMB Jena (available on the internet under: http://www.imb-jena.de/RNA.html). The prediction programs used by these servers use the same strategy for structure prediction based on an algorithm for calculating the free minimal energy of polyribonucleotide molecules (see: Stiegler & Zuker (1981) Nucleic Acids Res. 9, 133-48). While the different online resources may apply different optimization packages of this algorithm, analysis results obtained using the mRNA sequence of an MSP-1 p83/30 fragment subjected to structure prediction by different servers did not deviate from one another significantly with respect to the predicted secondary structure of the RBS. Thus, any of these programs/algorithims may be equivalently used with comparable results. In the online resources mentioned above, default values may be used for the prediction of RNA secondary structure. These default values are designed to reflect conditions in vivo. In contrast to parameters for the prediction of secondary structure in DNA, parameters for RNA folding cannot be adjusted with the user interface of the "mfold" server.

As used herein in this and other aspects/embodiments, the term "destabilize" or "destabilization" and the like refer to a decrease in the stability, and therefore persistence of, secondary structure such as hairpin-like structure in mRNA as compared to the persistence of secondary structure in the same region when no nucleotide modifications have been carried out. For example, modifying the nucleic acid sequence such that hybridization between two respective nucleotides or nucleotide stretches is disrupted and would be less likely to occur in a modified state as compared to an unmodified state would constitute a "destabilizing". As such, "destabilization" is not just the result of a lower free energy of the predicted molecule. Rather, it focuses on the secondary structure of the ribosomal binding site, with any involvement in a defined secondary structure being more "stable" than an unstructured region. For example, and while not being bound by theory, the present inventors postulate that part of a ribosomal binding site which, in an unmodified sequence, participates in a hairpin-like structure will be less accessible for binding by the ribosome than it will be in a sequence modified so as to reduce the degree of defined structure in this region.

As used herein in this and other aspects/embodiments, the term "hairpin-like region" denotes a region in which a first subregion of mRNA in the 5' → 3' direction forms a stably hybridized structure with a second subregion of mRNA in the 3' → 5' region, where the first and second subregions each form part of the same contiguous strand of mRNA. In a typical "hairpin" or "stem-loop" structure, very few nucleotides, typically 5-6 exist between the hybridized first and second subregions participating in the hairpin. In contrast, there is no particular limit to the number of nucleotides which may exist between the first and second hybridized subregions of the "hairpin-like region" of the coding mRNA for the MSP-1 p83/30 fragment. As can be seen in appended Fig.1, very many nucleotides may exist between the first and second subregions of the mRNA participating in the hairpin-like region.

A further aspect of the invention provides a composition comprising the above MSP-1, said MSP-1 comprising
a) a purified fragment p83/30 of the gp190/MSP-1 from *Plasmodium* without heterologous sequences, and
b) a purified fragment p38/42 of the gp190/MSP-1 from *Plasmodium* without heterologous sequences.

According to one embodiment, the components (a) and (b) in the inventive composition are preferably present in equimolar amounts, e.g. molar ratios of from 1.5:1 to 1:1.5, more preferably from 1.2:1 to 1:1.2 and most preferably from about 1:1 of component (a) to component (b). Preferably, at least 70%, more preferably at least 80% and most preferably at least 90% of the fragments in the composition are present as non-covalently associated dimer.

In a preferred embodiment, the composition of the invention has a purity of at least 95% and, more preferably, of at least 97.5%, as determined by SDS gel electrophoresis and silver staining. As used in this and other aspects and embodiments, the term "purity" refers to the absence of heterologous polypeptides, i.e. non-MSP-1 polypeptides, e.g. isolation tags.

In a further preferred embodiment, the composition has a content of degradation products of less than 30%, more preferably of less than 20% and most preferably of less than 15%, as measured by SDS gel electrophoresis and immuno-staining. In this context, the term "degradation products" refers to polypeptide molecules which result from a degradation of the p83/30 fragment or the p38/42 fragment as described above.

A further aspect of the present invention provides a pharmaceutical composition comprising the above MSP-1, said MSP-1 comprising
a) a purified fragment p83/30 of the gp190/MSP-1 from *Plasmodium* without heterologous sequences, and
b) a purified fragment p38/42 of the gp190/MSP-1 from *Plasmodium* without heterologous sequences.

In one embodiment the pharmaceutical composition may additionally comprise pharmaceutically acceptable carriers, diluents and/or adjuvants.

A further aspect of the invention provides a modified nucleic acid encoding the p83/30 subunit of MSP-1 from *Plasmodium* having an amino acid sequence as set out in SEQ ID NO: 1, 2 or 3, wherein the modified nucleic acid is obtainable by the above method.

A further aspect of the invention provides a vector comprising the modified nucleic acid as set out above.

In one embodiment, the vector is a polynucleotide vector, for example a plasmid, for example a plasmid of the pZ series (Lutz & Bujard (1999) Nucleic Acids Res. 25, 1203-10). Other suitable plasmid vectors are known to one of ordinary skill in the art. It is preferred that the polynucleotide vector further comprises a promoter operably linked to the modified nucleic acid encoding the p83/30 fragment of MSP-1 so as to drive transcription thereof. In another embodiment the polynucleotide vector may comprise an inducible promoter such as P_{A1lacO-1}.

In another embodiment, the vector is a viral vector, for example a vaccinia viral vector, for example modified vaccinia vector Ankara (MVA), an adenovirus such as adenovirus serotype 5, or a measles virus such as an attenuated strain from Edmonton isolate. As is well known to the skilled person, viral vectors such as MVA are suitable for manufacturing safe genetic vaccines in which a nucleic acid or nucleic acids encoding an antigenic polypeptide(s) intended to immunize a subject is/are introduced into the body of subject, where the antigenic polypeptides are then expressed, recognized by the subject's immune system, and lead to the mounting of an innate and/or adaptive immune response against the expressed polypeptide(s). Alternatively, the vector may for example be attenuated Mycobacterium tuberculosis.

A further aspect of the invention provides a method of preparing the p83/30 fragment of *Plasmodium* MSP-1, said method comprising:
a) Providing a modified nucleic acid as set out above and/or a vector as set out above;
b) Incorporating the modified nucleic acid and/or vector of step (a) into a cell, preferably a prokaryotic cell;
c) Incubating the cell of step (b) under conditions allowing expression of the p83/30 fragment of *Plasmodium* MSP-1; and
d) Recovering the p83/30 fragment of *Plasmodium* MSP-1.

The inventors have determined that using a p83/30-encoding nucleic acid sequence modified as described hereinabove (e.g. as set out in SEQ ID NO: 8 or 9) leads to greatly increased expression of the p83/30 fragment of *Plasmodium* MSP-1, allowing very efficient production of this protein fragment within the host cell. Without being bound by theory, the inventors believe that the rapid increase in protein levels in a host transfected with the modified nucleic acid sequence as set out above leads to rapid aggregation and precipitation of this protein as inclusion bodies before the translated protein can be subjected to intracellular proteolytic degradation. Accordingly, it is believed that the p83/30 protein produced is rapidly converted into inclusion body form, where the protein remains protected from degradation by endogenous proteolytic enzymes of the host cell.

Without being bound by theory, the inventors thus believe that the amounts of p83/30 fragment protein obtained upon recovery are greater than previously possible for two reasons: First, the use of modified nucleic acid as set out above (e.g. SEQ ID NO: 8 or 9) enables more efficient recognition by ribosomes and initiation of translation than possible for unmodified nucleic acid, where ribosomal recognition sites remain buried in regions of secondary structure. This enables translation to begin more efficiently. Second, once translation of the p83/30 subunit is complete, this protein appears to be converted rapidly to inclusion bodies, where it remains protected from degradation by the host cell until it is recovered.

As used herein in this and other aspects/embodiments, the term "conditions allowing expression" denote any environmental conditions known to the skilled person to growth and/or promote protein expression from a chosen host expression system, i.e. a chosen strain of host cell. These conditions may include for example, temperature, light, shaking speed, pH of the medium, nutrient composition of the medium as well as the presence or absence of growth factors, amino acids, vitamins and the like known to promote protein expression in the type of host cell chosen. For example, using *E. coli* as a host cell for the heterologous preparation of the p83/30 fragment of *Plasmodium* MSP-1, suitable conditions may be as set out in the appended Examples, e.g. Example 3.

In one embodiment, the recovering step comprises solubilizing the MSP-1 protein subunit from inclusion bodies formed within the host cell. As used herein in this and other aspects/embodiments, the term "inclusion bodies" refers to protein aggregates in the cytoplasm of bacteria that are formed by heterologously expressed genes. For many recombinant proteins the environment in the prokaryotic cytoplasm is not suitable for proper protein folding, particularly when the protein is present at high concentration due to overexpression. The proteins precipitate and form large protein aggregates termed "inclusion bodies" that can be identified microscopically as high molecular weight structures inside the bacteria. Protein in the inclusion bodies is usually not present in its natural conformation and must therefore be refolded *in vitro* from inclusion bodies before it can function properly. As the information for refolding is contained in the amino acid sequence of a protein, refolding procedures differ for each protein and need to be established in detail on a case-by-case basis, as dictated by the particular protein expressed and present as inclusion bodies. Nevertheless, certain basic parameters have been identified which are advantageous for the folding of a wide variety of proteins (see for example Rudolph et al. (1997) Protein function: A practical approach. Ed. T.E. Creighton). One of ordinary skill in the art understands how to apply these general parameters (e.g. choice of renaturation buffers) to the isolation and refolding of protein in the form of inclusion bodies.

The inventors have found that one particularly efficient method for renaturing solubilized inclusion bodies of MSP-1 proteins (both p83/30 and p38/42 fragments thereof) involves pulsing the solution containing the solubilized inclusion bodies. In this sense, "pulsing" refers to the addition of the respective protein solution into a renaturation buffer in which refolding is to take place. This process is termed "pulse renaturation" and has been described previously (Rudolph & Lillie (1996) FASEB J. 10, 49-56). Briefly, small amounts of denatured proteins are added (i.e. "pulsed") into a renaturation buffer in separate aliquots, spaced by time intervals, e.g. at least one hour. During a respective interval, protein folding can occur at low concentrations, thereby decreasing the probability of aggregation. For example, instead of adding 1 mg of protein to the renaturation solution all at once, it can be advantageous to add the protein gradually, in smaller amounts or "pulses", for example stirring shortly before, during and shortly following a respective pulse. In this way, for example, 10 individual additions of 0.1 mg of protein each may be added over, for example, 10 hours; one pulse per hour briefly preceded and followed by stirring, and with the solution being allowed to incubate at rest prior to the next respective addition of protein. Refolded protein does not interfere with the renaturation of additional protein which has not yet refolded.

In the renaturation of p83/30 and p38/42 fragments of *Plasmodium* MSP-1, the inventors have found it particularly advantageous to add protein in intervals of about one hour, a time span which has been previously shown to allow a wide variety of proteins to refold. For the application, or "pulsing", of protein, any device allowing the addition of a defined volume of solution may be used, e.g. automated pipettor. The inventors have found it to be particularly advantageous to stir the solution about one minute before, during and about one minute after a respective protein addition. These procedure settings, as well as the approximate one hour time interval between respective protein additions, have been previously described (Rudolph & Lillie (1996) FASEB J. 10, 49-56).

Such pulse-renaturation techniques are especially applicable to large scale preparatory processes of the type needed for preparing the large amounts of MSP-1 subunits required for the manufacture of vaccines. Further, in the event that host cells are transfected with nucleic acid encoding only the p83/30 MSP-1 protein subunit, then the inclusion bodies should contain only this protein, meaning that it can be efficiently isolated from the cell lysate by standard physical separation techniques (such as centrifugation). The high-yield, inclusion body-forming process developed and implemented by the inventors thus allows efficient isolation of the p83/30 MSP-1 protein subunit in a way which completely avoids any dependence on isolation tags.

A further aspect of the invention provides a p83/30 subunit of the MSP-1 protein obtainable by the corresponding preparation method set out above. As explained above, previous attempts to prepare amounts of MSP-1 protein fragment p83/30 sufficient for technical scale preparation of a vaccine failed due to a) low heterologous expression levels and b) proteolytic degradation by the host cell of the small amounts expressed. The vastly improved yields of MSP-1 subunit p83/30 of the present invention are the result of the inventors' recognition of which types of modifications need to be effected in nucleic acid encoding this protein subunit. These modifications allow more efficient, rapid expression of the p83/30 protein product. The rapid production of this protein in host cells leads to the accumulation of inclusion bodies, in which form the p83/30 subunit remains protected from degradation by the host cell. Since the p83/30 subunit is present in large quantities in easily separable inclusion bodies, it is isolable in highly pure form using standard physical separation techniques such as centrifugation, avoiding the need to rely on isolation tags for protein recovery.

The inventors' recognition of the need for specific expression-enhancing modifications in the nucleic acid encoding the p83/30 subunit of MSP-1 thus in effect eliminates the one step which has hitherto hampered technical scale preparation of MSP-1 heterodimers comprising this subunit. At the same time, GMP-compliant protein is obtained which can be submitted to the appropriate regulatory agencies for approval as an administrable protein therapeutic.

A further aspect of the invention provides a method of preparing a *Plasmodium* MSP-1 containing composition, said method comprising:
a) Expressing the fragment p83/30 of the gp190/MSP-1 protein from *Plasmodium* without heterologous sequences in a host cell;
b) Expressing the fragment p38/42 of the gp190/MSP-1 protein from *Plasmodium* without heterologous sequences in a host cell;
c) Recovering the fragment p83/30 and the fragment p38/42 from the host cells; and
d) Optionally combining the fragments p83/30 and p38/42 in a composition or as a combined preparation.

The expression of the fragments p83/30 and p38/42 may be carried out in a single host cell or separately in a first host cell and in a second host cell. The use of separate first and second host cells is preferred. The host cell may be provided by transfection with a nucleic acid encoding the respective MSP-1 fragment. Preferably, the nucleic acid encoding the p83/30 fragment and the nucleic acid encoding the p38/42 fragment may be located on an expression vector suitable for the respective host cell, e.g. a gram-negative bacterial cell such as an *E. coli* cell. The expression vector may be an episomal vector such as a plasmid, or a chromosomally integrated vector. The expression vector comprises the nucleic acid in operative linkage with a suitable expression control sequence, e.g. which may comprise a constitutive or an inducible promoter. The expression vector may comprise further genetic elements, e.g. an origin of replication, a selection marker, etc. Examples of suitable cloning vectors are disclosed in Sambrook et al., Molecular Cloning, A Laboratory Manual (1989), Cold Spring Harbor Laboratory Press, and other standard textbooks.

When the host cells are bacterial cells, e.g. *E. coli* cells, the fragments are preferably expressed in insoluble form, e.g. as inclusion bodies, which are discussed hereinabove. Preferably, the fragments are separately recovered as inclusion bodies which may be separately solubilized, e.g. in the presence of chaotropic salts such as guanidinium hydrochloride and subsequently refolded, e.g. in the presence of arginine and thiol reagents such as glutathione. Alternatively, the p83/30 and p38/42 fragments may be combined before solubilization, after solubilization or after refolding in a suitable molar ratio. The pulsed renaturation techniques discussed hereinabove may be used in this regard. After refolding, the fragments are transferred to a suitable buffer which may comprise a non-ionic surfactant such as Tween 80, Tween 20 or Triton X-1 00. If desired. The fragments may be separately purified by subsequent processing steps, comprising at least one of the following: filtration, anion and/or cation exchange chromatography such as Q-sepharose HP-chromatography, or SP-sepharose HP-chromatography, conditioning and concentration, e.g. by ultrafiltration.

According to step (d), the fragments are combined. Preferably, the fragments are combined in about equimolar amounts as indicated above. The amount of the respective fragment may be determined by quantitative SDS-PAGE performed under reducing conditions. It is especially preferred that the fragments are combined in the absence of heterologous polypeptides such as albumin.

After combining, the fragments may be further purified, e.g. by size exclusion chromatography, e.g. using Sephacryl S 300-HR/GE. Further treatments may comprise filtration, concentration and sterilization, e.g. by sterile filtration.

The nucleic acids encoding the fragments p83/30 and p38/42 may have reduced AT-content compared to the wild type sequence as described in WO 98/14583, which is herein incorporated by reference.

Generally, efficient production of the p38/42 fragment, e.g. having an amino acid sequence as set out in SEQ ID NO: 4 or 5 does not suffer from the same problems of low yield observed when producing the p83/30 fragment from unmodified nucleic acid. As used herein, "unmodified nucleic acid" denotes nucleic acid which, with respect to the coding sequence for the individual MSP-1 subunits p83 and p30 in a single polypeptide chain, corresponds to the native nucleic acid sequences (or native nucleic acid sequences which have been previously modified to increase stability as for example disclosed in WO 98/14583 and Pan et al. (1999) Nucleic Acids Res. 27(4), 1094-103) for the respective subunits in *Plasmodium,* preferably in *P. falciparum.* Generally, it should thus be possible to use a nucleic acid comprising native, unmodified sequences encoding the p38 and p42 subunits to efficiently produce the MSP-1 fragment p38/42. However, corresponding modification of the nucleic acid encoding the p38/42 fragment of MSP-1 may be carried out in a fashion analogous to that described above for the p83/30 fragment if desired, expedient or necessary. The advantages described above for the p83/30 fragment of MSP-1, for example efficient production in the form of inclusion bodies and omission of any heterologous sequences, e.g. isolation tags, thus also apply to the p38/42 subunit as well.

The inventors have determined that the two MSP-1 subunit fragments p83/30 and p38/42 spontaneously refold and associate under suitable conditions. This is especially the case when the fragments have previously been resolubilized out of inclusion body form. These conditions are described in detail herein. As such, it is possible to efficiently construct heterodimeric MSP-1 protein comprising the synthetic MSP-1 subunits p83/30 and p38/42 in a single concerted process without the need for prior yield-lowering refolding procedures for the individual subunits. The yield of the resulting MSP-1 protein is thus maximized. Further, since refolding and reassembly of MSP-1 take place spontaneously, traditional column-based protein isolation procedures may in most cases be dispensed with and, with them, the need for isolation tags of any sort, as described hereinabove. Of course, such column purification methods as described herein above may still be employed if desired.

As used herein in this and other aspects/embodiments, "mixing" or "combining" denotes bringing the p83/30 and p38/42 subunit fragments of MSP-1 into contact with one another under conditions which promote heterodimer formation. Suitable conditions to this end are described hereinbelow.

One very advantageous feature of this aspect of the invention is that the two "halves" of MSP-1, i.e. the p83/30 and p38/42 fragments may be brought together as solubilized but non-refolded inclusion bodies to form complete MSP-1. Thus this embodiment avoids the need for separate refolding of the individual p83/30 and p38/42 subunits prior to mixing with one another. A solution containing both of the solubilized but still unfolded protein subunits may then be treated so that the individual subunits gradually renature or "refold" with one another, spontaneously reassembling the full, correctly folded MSP-1 heterodimer as they do. As indicated above, it is advantageous to combine the two subunits in stoichiometric, or near-stoichiometric amounts (i.e. in a ratio of 1:1 or close to 1:1). As such, the protein concentrations of the inclusion body preparations of the respective MSP-1 fragments should be determined in advance of mixing. The ability to reconstitute the MSP-1 heterodimer in one concerted step starting from still insoluble inclusion bodies thus eliminates any need to renature each of the subunits separately prior to mixing, a process which can incur product loss. As such, the possibility of mixing the individual protein subunits without any other prior treatment steps than recovery of inclusion bodies (and their optional prior solubilization) thus exploits the high protein yields obtained for the p83/30 subunit by appropriate nucleic acid modification and for the p38/42 by using the native or modified *Plasmodium* sequences to the maximum extent possible.

In one embodiment, the constituent p83/30 and p38/42 subunits of the MSP-1 heterodimer may be incorporated into the same or different cells, preferably prokaryotic cells. However, It is preferred in one embodiment that the combining step above is performed such that the p83/30 and p38/42 subunits are mixed in an approximate stoichiometric ratio of 1:1 in relation to one another. To this end, it is preferred that the p83/30 and p38/42 subunits of the MSP-1 heterodimer be expressed from separate cells. This enables a greater degree of experimental control in adjusting the stoichiometric ratio in which the protein subunits are later mixed such that this ratio is as close to 1:1 as possible.

In one embodiment, the individual subunit inclusion bodies are solubilized separately, i.e. in separate solutions prior to mixing for reassembly. One can then determine their respective amounts in solution, and combine appropriate amounts of each solution to reconstitute an entire MSP-1. In one preferred embodiment, the two solutions containing the p83/30 and p38/42 subunits can be added as metered aliquots, i.e. "pulses" into one mixture solution, as discussed above. In one particularly preferred embodiment, the inventors have found that the reconstitution, i.e. reassembly of the MSP-1 heterodimer from its respective p83/30 and p38/42 halves may be promoted by timing the respective additions in the approximate middle of stirring periods lasting about 2 minutes. That is to say, following a respective incubation during which protein renaturation has been allowed to take place, the solution is gently stirred for about 1 minute. Stirring continues during protein addition, and stirring is continued for about one further minute. After this period, stirring is stopped and the solution is allowed to incubate at rest for a predetermined time period, advantageously. Following this rest period, the process is repeated until all available protein has been added.

In another embodiment, the p83/30 and p38/42 subunits are not renatured prior to the mixing step, but rather are added to one another in denatured form, as already described above. For example, the denatured form may include inclusion bodies which have been recovered from a cell lysate by centrifugation and resuspended (but not renatured) prior to the mixing step. In one embodiment, the mixture solution containing denatured p83/30 and p38/42 is subjected to the same type of pulse treatment as described above.

In another embodiment, one of the two protein subunits of the MSP-1 heterodimer is refolded prior to being mixed with the other of the two subunits, the latter being still denatured and soluble or insoluble upon mixing. Here, as above, a pulsing process may be employed to solubilize the as yet unsoluble subunit (if needed), and simultaneously promote association of the two protein subunits into correctly folded MSP-1.

In one embodiment, actual recovery of the MSP-1 heterodimer can be accomplished for example by diafiltration or dialysis with salt exchange to any buffer desired without the need of including stabilizing components such as BSA or HSA. A range of different buffers may be used, as required for subsequent purification and formulation processes.

A further aspect of the invention provides an MSP-1 from *Plasmodium* as set out above, the modified nucleic acid as set out above, the vector as set out above, the MSP-1 fragment p83/30 as set out above (e.g. any of SEQ ID NO: 1, 2 or 3) or an MSP-1 fragment p38/42 (e.g. any of SEQ ID NO: 4 or 5) for use as a vaccine.

As mentioned above, the fully reconstituted MSP-1 heterodimer as well as the individual p83/30 and p38/42 protein subunits thereof are capable of eliciting an immune response when brought into contact with the immune system of a host, i.e. the immune system of an animal or a human. Especially preferred to this end is the use of the MSP-1 heterodimer comprising both the p83/30 and p38/42 subunits, as the heterodimer will present the host immune system with epitopes most closely matching those of the native MSP-1 heterotetramer. However, also preferred is the use of either of the protein subunits p83/30 or p38/42 to this end, as these will present the host immune system with certain epitopes equivalent to those in heterodimeric MSP-1.

In one embodiment, the vaccine comprises a pharmacologically compatible carrier, i.e. adjuvant. Pharmacologically compatible adjuvants in this respect are all carriers and agents known in the art to enhance the immune response. Preferred adjuvants in this regard include aluminium oxide and aluminum phosphate carriers, each with or without additional stimulating compounds such as MPL, ISA 720 and ISA 51 from Seppich SA, IC31 from Intercell or MF59.

In one embodiment, the vaccine is suitable for administration by injection, for example intradermal, intramuscular or subcutaneous. In general, however, any route of administration known to lead to an immune response in vaccine administration may be used to administer the inventive vaccine.

The vaccine may be prepared as a reconstitutible lypphilisate, as a liquid, e.g. as a solution or a suspension, or as an emulsion, e.g. as a water-in-oil emulsion. A preferred dosage of the vaccine comprises 1-500 µg, more preferably 20-100 µg for application in human medicine. The vaccine may be administered in a single dose or in multiple doses. The administration in multiple doses is preferred. Doses of 100 µg are especially preferred.

A further aspect of the invention provides a use of MSP-1 from *Plasmodium* as set out above, the modified nucleic acid as set out above, the vector as set out above, the MSP-1 fragment p83/30 as set out above (e.g. any of SEQ ID NO: 1, 2 or 3) and/or an MSP-1 fragment p38/42 (e.g. any of SEQ ID NO: 4 or 5) in the manufacture of a medicament for preventing and/or treating malaria.

In one embodiment, the medicament is prepared for administration together with an adjuvant, wherein exemplary adjuvants are as set out above. Exemplary routes and amounts of administration are also as set out above.

In a preferred embodiment, the medicament is prepared for administration together with ISA720. The inventors have surprisingly found that the administration of MSP-1 comprising the protein subunits p83/30 and p38/42 (each without heterologous sequences) to animals leads to an unusually high antibody titer, as evidenced by a) comparison with titers obtained using Freund's complete/incomplete adjuvant, the most potent adjuvant known, and b) examination of the specificity of the antibodies obtained (high titers of antibodies were obtained also against the low immunogenicity regions p30 and p38). Thus, the modifications made in the p83/30-encoding nucleic acid not only allow production of the corresponding subunit for the first time in acceptable yield and free of any isolation tags, but also the finding of a beneficial antigen/adjuvant combination resulting in a promising formulation of MSP-1 from *Plasmodium,* together with the adjuvant ISA720.

The embodiments above in the context of pharmaceutically compatible carriers/adjuvants/diluents as well as administration routes and amounts apply to this aspect of the invention *mutatis mutandi*.

A further aspect of the invention provides a vaccine comprising MSP-1 from *Plasmodium* as set out above, the modified nucleic acid as set out above, the vector as set out above, the MSP-1 fragment p83/30 as set out above (e.g. any of SEQ ID NO: 1, 2 or 3) and/or the MSP-1 fragment p38/42 (e.g. any of SEQ ID NO: 4 or 5).

A further aspect of the invention refers to a composition comprising a purified fragment p83/30 of the gp190/MSP-1 protein from a *Plasmodium* F-strain. The p83/30 fragment preferably comprises the sequence as shown in SEQ ID NO: 3 or a modified sequence as described above. This composition is preferably used as a pharmaceutical preparation, e.g. as a vaccine as described above. With regard to the preferred characteristics, e.g. purity and/or content of degradation products, of this composition, reference is made to the disclosure as described above.

The embodiments above in the context of pharmaceutically compatible carriers/adjuvants/diluents as well as administration routes and amounts apply to this aspect of the invention *mutatis mutandi*.

The invention will now be illustrated by way of the following non-limiting examples.

### Examples

### Example 1: Mutation of nucleic acid sequences encoding MSP-1 protein subunits

In prokaryotes the ribosome binding site, also referred to as "Shine-Dalgarno-sequence", consists of purine-rich sequences approximately 10 nucleotides upstream of the start codon. The ribosomal binding site sequence in the plasmid pZE23/d-83/30 used for the expression of the recombinant MSP-1 proteins corresponds to the "Shine-Dalgano" consensus sequence (Shine & Dalgamo (1975) Nature 254, 34-8) and its capacity of driving gene expression in *E. coli* had been described previously (Lutz & Bujard (1997) Nucleic Acids Res. 25, 1203-10). Due to the fact that in prokaryotes translation of mRNA into protein takes place while transcription is still ongoing, the secondary structure of the mRNA might influence the recognition of the mRNA by ribosomes and, as a consequence, the rate of protein synthesis.

In light of the above, *in silico* analysis of the mRNA structure was performed by submitting about the first 150-200 bp of the mRNA encoding p83/30 (i.e. the first 150-200 bp of either of SEQ ID NO: 6 or 7) to the RNA structure prediction program "mfold" (version 3.2) provided as an online resource by Rensselaer Polytechnic Institute, Troy, NY (Zuker (2003) Nucleic Acids Res. 31 (13), 3406-15; http://www.bioinfo.rpi.edu/applications/). Default values were used for the prediction of RNA secondary structure. These default values are designed to reflect conditions *in vivo*. In contrast to parameters for the prediction of secondary structure in DNA, parameters for RNA folding cannot be adjusted with the user interface of the "mfold" server. SEQ ID NO: 6 and 7 are shown in Figs. 15 and 16, respectively.

The predicted structure of the ribosomal binding site was found to contain a hairpin-like structure, which might restrict access of ribosome (see Fig.1). It was hypothesized that by modifying the secondary structure of the mRNA such that the ribosomal binding site is no longer part of a structurally well-defined region, such as the hairpin-like structure, ribosomal access to the mRNA might be facilitated, thus promoting synthesis of recombinant MSP-1 in the bacteria.

To this end, several mutations were introduced into sequences interacting with the ribosome in order to destabilize the hairpin-like structure. The modified sequences were again subjected to computational analysis and showed the expected effect of the mutations, i.e. the structure of the ribosomal binding site was no longer part of a hairpin-like structure.

Following this computational prediction, two different sets of the computed mutations were introduced by site-specific mutagenesis into the p83/30-encoding nucleic acids. Sequences with either set of mutations as well as a sequence containing both sets of mutations were finally cloned and all lead to improved MSP-1 expression as compared to the original sequence. The sequence containing both sets of mutations proved to be most effective and was then used for the expression of p83/30. Both sets of mutations are present in SEQ ID NO: 8 and 9 in identical form (see Figs 17 and 18). These mutations relative to the parental nucleic acid sequences of SEQ ID NO: 6 and 7, respectively, are silent mutations, meaning that they do not lead to modifications at the amino acid level. The nucleotides in SEQ ID NO: 8 and 9 which were mutated relative to SEQ ID NO: 6 and 7 are emphasized in respective SEQ ID NO: 8 and 9 in **lower case boldface**, and the start codon ATG is underlined (see Figs. 17 and 18).

### Example 2: Comparison of expression of non-mutated with mutated MSP-1 subunits.

*E. coli* W3110-Z2 containing pZE23/d-83/30 (unmodified sequence for the p83/30 fragment, corresponding to SEQ ID NO: 6 or 7, in which the ribosomal binding sites are identical) or pZE23/d-mut83/30 (modified sequence for the p83/30 fragment, corresponding to SEQ ID NO: 8 or 9, in which the ribosomal binding sites have been identically modified) were grown in Superbroth medium at 37°C to a cell density of OD500 = 0.5. MSP-1 expression was induced by addition of IPTG (1 mM) and bacteria were grown for an additional 4 hours. Aliquots of whole bacterial extracts were taken at the time of induction (0 h) and after 2 (2h) and 4 hours (4h), respectively. Whole cell extracts were analyzed by SDS-PAGE and stained with Coomassie. The results obtained are shown in Fig. 2.

### Example 3: Production of heterodimeric MSP-1 from p83/30 and p38/42

### Example 3a: Production of host cells with translationally optimized p83/30 and p38/42

CaCl₂-competent *E. coli* W3110Z2 are transformed with plasmids pZE23/mut-d-83/30 and pZE23/d38/42. *E.coli* host strains W3110Z2-pZE23/d83/30 and W3110Z2-pZE23/d38/42 are grown separately, but under identical conditions: in Superbroth at 37°C. At cell densities of OD600 = 7, synthesis of the MSP-1 subunits is induced by addition of Isopropyl beta-D-1-thiogalactopyranoside ("IPTG"; final concentration 1 mM). At OD = 18-24 cells are harvested by centrifugation and either stored at - 80°C or immediately processed further. The results are shown in Fig. 3 for the expression of p83/30 and p38/42 fragments from mutated and non-mutated nucleic acid sequences, respectively.

### Example 3b: Expression of p83/30 and p38/42 subunits

Fermentations of the two *E. coli* host strains W3110Z2-pZE23/d83/30 and W3110Z2-pZE23/d38/42 were carried out separately, but under identical conditions: in Superbroth at 37°C. At cell densities of OD600 = 7 synthesis of the MSP-1 fragments was induced by addition of IPTG (final concentration 1 mM). At OD = 18-24 cells are harvested by centrifugation and either stored at - 80°C or immediately processed further. The results of the fermentation are shown in Fig. 4.

### Example 3c: Recovery and solubilization of p83/30 and p38/42 subunits from inclusion bodies

Cells from fermetation or from frozen biomass were suspended in lysis buffer at 4°C and opened via a fluidizer at 1.034x10⁵ - 1.379x10⁵ kPa (15,000 - 20,000 psi). Centrifugation at 27,000 g was performed for 20 min. The pellet contained the inclusion bodies. The pellet was resuspended in lysis buffer (PBS, pH 7.4, lysozyme, Dnase), and centrifugation was repeated. The pellet was harvested and subjected to another cycle of resuspension and centrifugation. The final concentration of inclusion bodies was 500 mg/L bacterial culture. The inclusion bodies collected in the pellet were dissolved in solubilization buffer (6 M guanidinium hydrochloride, 50 mM sodium phosphate, pH 8.0, 10 mM DTT, 1mM EDTA) while stirring at room temperature for 10 hours. Centrifugation at 27,000 g for 40 min cleared the solution of dissolved inclusion bodies. Fig. 5 shows electrophoretic analysis of solubilized inclusion bodies.

### Example 3d: Mixing and renaturation of p83/30 and p38/42 to form reassembled MSP-1 heterodimer

The content of the MSP-1 fragments p83/30 and p38/42 in their respective solubilized inclusion body preparations was determined by electrophoretic analysis of a dilution series of both preparations. These determinations are important for calculating the volumes of the two preparations to be combined during the pulse renaturing process. Both subunits were added to the renaturation buffer (500 mM arginine, 50 mM sodium phosphate, pH 8.0, 1 mM EDTA, 0.1 mM glutathione) in ten equal aliquots, such that the final concentration was 0.5 mg/mL renatured heterodimer at the end of the renaturation process.

The individual aliquots were added to the renaturing buffer in the following way: The solution was stirred for 1 min before, during and after each addition, i.e. "pulse" of protein. About 1 hour was allowed to elapse between stopping stirring following one addition and starting stirring prior to the next respective addition. Following the last addition, the solution was incubated for an additional 10 hrs. The entire process was temperature controlled at 10°C.

Following incubation, the protein solution was centrifuged or diafiltered at 4°C. The buffer was exchanged by diafiltration. Protein recovery was 99%.

### Example 3e: Purification of the reconstituted MSP-1 heterodimeric protein

The renatured MSP-1 heterodimer comprising both the p83/30 and p38/42 fragments without heterologous polypeptide sequences such as isolation tags was subjected to three chromatographic fractionation processes: HP-Q sepharose chromatography (AP Biotech), hydroxyapatite chromatograohy, macroprep Type I (BioRad), and HP-SP sepharose chromatography (AP Biotech).

The overall yield and purity of the MSP-1 Heterodimer per 100 L fermentation volume was 8 gm of MSP-1. This is an amount of MSP-1 sufficient for 80,000 doses of 100 µg each. The end product contained less than 1 ng of *E. coli* protein per 1 mg of MSP-1.

About 85% of the final material consists of intact MSP-1 heterodimeric protein. The end-product contained about 15% of so-called product-derived impurities. These impurities are the product sensitivity to protease. Of the two fragments p83/30 and p38/42, it was primarily the p83/30 subunit which showed such sensitivity to protease. The degradation products (see Fig. 6) appear to constitute parts of incomplete heterodimers, as they cannot be separated from the complex. All detectable components of the purified MSP-1 preparation can be identified as degradation products of MSP-1 (Fig. 6), as determined using appropriate antibodies specific for the p83/30 and p38/42 fragments of MSP-1.

### Example 4: Immunogenicty studies

Immunogenicity of an antigen strongly depends on (1) the way it is formulated, i.e. with which adjuvant it is combined; (2) the immunization regimen, i.e. the way it is administered (for example, subcutaneously, s.c. or intramuscularly, i.m.); (3) the number of immunizations carried out; and (4) the temporal sequence of the immunizations.

In the present study, examination of adjuvants was limited to those which are suitable for use in humans. Freud's complete and incomplete adjuvant (FCA, FIA), a very potent adjuvant not accepted for use in humans, was used as a positive control. A further control was pure antigen in phosphate buffered saline (PBS).

A first set of immunizations was carried out in a mouse model. Antigen/adjuvant combinations which showed a high immunogenicity in these animals as verified by high titers of MSP-1-specific antibodies were subsequently examined in rabbits.

### Example 4a: Immunization in Balb c mice

Groups of seven animals were injected with the antigen/adjuvant combination containing 5 µg of antigen per dose. Three subcutaneous injections were performed on days 0, 21 and 42. On day 63, animals were bled and final serum was harvested. Antibody titers were determined via ELISA using MSP-1 heterodimer as capture protein.

Immunizations were carried out with the following adjuvants: aluminum hydroxide; aluminum phosphate; MPL (Sigma); CpG (Qiagen); IC31 (Intercell, Vienna, Austria); Montanide ISA 720 (Seppich, Paris, France); Montanide 51 (Seppich); Virosomes (Bema Biotech, Bern, Switzerland); and FCA/FIA.

Results of this study are shown in Fig.7. The identities of the individual clusters of the data bars A-Y are as set out in the corresponding description of Fig. 7 hereinabove. Importantly, data cluster A represents antigen administered in PBS, data cluster A represents antigen administered in FCA/FIA. As can be seen in Fig.7, data clusters K (corresponding to the adjuvant X3), S (corresponding to the adjuvant Montanide ISA720) and T (corresponding to the adjuvant Montanide ISA51) resulted in the highest antibody titers of all test formulations. Based on these results, MSP-1 and MSP-1-related antigen was subsequently administered with each of these three adjuvants to rabbits.

### Example 4b: Immunization of rabbits

Groups of five rabbits were injected intramuscularly with four doses of formulated antigen (100 µg per dose). Immunizations were performed on days 0, 28, 42 and 56. On day 70 animals were bled and sera were harvested. The titer of MSP-1-specific antibodies was measured via ELISA using MSP-1 heterodimer as well as the 4 subunits p83, p30, p38, and p42 as capture proteins. Results are shown in Fig. 8.

As Figs. 7 and 8 each show, there exist several promising antigen/adjuvant combinations and further studies are likely to reveal additional candidates. Present data indicate, however, that the combination of MSP-1 heterodimer with Montanide ISA 720 is particularly promising, for two surprising reasons:
(1) The antibody titer obtained in rabbits surpasses the combination of MSP-1 with Freund's adjuvant.
(2) The titer of antibodies when analyzed using the four subunits of MSP-1 show a most interesting profile. Titers against p30 and p38 are unusually high in comparison to those specific for p83 and p42. As the two subunits p30 and p38 elicit antibodies which are particularly effective in inhibiting parasite growth, the outcome of this study is particularly promising.

## Claims

1. A merozoite surface protein 1 ("MSP-1") preparation of *Plasmodium falciparum,* said MSP-1 comprising
(a) a purified fragment p83/30 of the gp190/MSP-1 from *Plasmodium* without heterologous sequences, and
(b) a purified fragment p38/42 of the gp190/MSP-1 from *Plasmodium* without heterologous sequences.

2. The MSP-1 preparation of claim 1, wherein the *Plasmodium* is the 3D7 strain of *Plasmodium falciparum.*

3. The MSP-1 preparation of claim 1 or 2, wherein the MSP-1 preparation is provided as a heterodimer comprising the MSP-1 fragments p83/30 and p38/42.

4. The heterodimeric MSP-1 preparation of claim 3, wherein the MSP-1 protein subunit p83/30 comprises an amino acid sequence as set out in SEQ ID NO: 1, 2 or 3 and the MSP-1 protein subunit p38/42 comprises an amino acid sequence as set out in SEQ ID NO: 4 or 5.

5. A method for preparing a modified nucleic acid encoding the p83/30 subunit of the MSP-1 from *Plasmodium* having an amino acid sequence as set out in SEQ ID NO: 1, 2 or 3, said method comprising:
a) Providing an mRNA sequence encoding the p83/30 subunit of the MSP-1 protein from *Plasmodium* having an amino acid sequence as set out in SEQ ID NO: 1, 2 or 3;
b) Subjecting the region surrounding the start codon of the mRNA sequence of step (a) to analysis *in silico* to determine the predicted secondary structure of said mRNA sequence;
c) Identifying at least one stable, hairpin-like structure in said region surrounding the start codon of step (b);
d) Modifying the sequence of the mRNA in said region surrounding the start codon of step (b), wherein at least one nucleotide substitution, insertion, deletion and/or inversion destabilizes said hairpin-like region, and wherein the at least one nucleotide substitution, insertion, deletion and/or inversion is effected *in silico* so as to encode the same amino acid sequence as encoded by said unmodified nucleic acid sequence;
e) Subjecting the region of step (d) to analysis *in silico* under the same conditions as set out in step (b);
f) Determining whether the at least one nucleotide substitution, insertion, deletion and/or inversion destabilizes the at least one hairpin-like region containing the at least one ribosomal binding site;
g) Optionally repeating steps (d) - (f) as needed until analysis *in silico* under the same conditions as set out in step (b) indicates the destabilization of the at least one hairpin-like region;
h) Preparing the corresponding modified nucleic acid.

6. A modified nucleic acid encoding the p83/30 subunit of the MSP-1 protein of *Plasmodium* having an amino acid sequence as set out in SEQ ID NO: 1, 2 or 3, wherein the modified nucleic acid is obtainable by the method of claim 5.

7. The modified nucleic acid of claim 6, wherein the nucleic acid comprises a nucleotide sequence as set out in any of SEQ ID NOs: 8 or 9.

8. A vector comprising the modified nucleic acid of claim 6 or 7.

9. The vector of claim 8, wherein the vector is a polynucleotide vector or a viral vector.

10. A method of preparing the p83/30 fragment of the MSP-1 from *Plasmodium,* said method comprising:
a) Providing a modified nucleic acid of claim 6 or 7 and/or a vector of claim 8 or 9;
b) Incorporating the modified nucleic acid and/or vector of step (a) into a cell, preferably a prokaryotic cell;
c) Incubating the cell of step (b) under conditions allowing expression of the p83/30 fragment of the MSP-1 protein; and
d) Recovering the p83/30 fragment of the MSP-1 protein.

11. The method of claim 10, wherein said recovering step comprises solubilizing said p83/30 subunit of the MSP-1 protein from inclusion bodies.

12. A p83/30 subunit of the MSP-1 protein obtainable by the method of claim 10 or 11.

13. A method of preparing a *Plasmodium* MSP-1 containing composition, said method comprising:
a) Expressing the fragment p83/30 of the gp190/MSP-1 protein from *Plasmodium* without heterologous sequences in a host cell;
b) Expressing the fragment p38/42 of the gp190/MSP-1 protein from *Plasmodium* without heterologous sequences in a host cell;
c) Recovering the fragment p83/30 and the fragment p38/42 from the host cells; and
d) Optionally combining the fragments p83/30 and p38/42 in a composition or as a combined preparation.

14. The method of claim 13, wherein the combining step (d) is performed such that the p83/30 fragment and the p38/42 fragment are mixed in an approximate stoichiometric ratio of 1:1 in relation to one another.

15. The MSP-1 preparation of any of claims 1-4, the modified nucleic acid of claim 6 or 7, the vector of claim 8 or 9, the MSP-1 fragment p83/30 of claim 12 and/or the MSP-1 fragment p38/42 having an amino acid sequence as set out in SEQ ID NO: 2 for use as a vaccine.

16. Use of the MSP-1 preparation of any of claims 1-4, the modified nucleic acid of claim 6 or 7, the vector of claim 8 or 9, the MSP-1 fragment p83/30 of claim 12 and/or the MSP-1 fragment p38/42 having an amino acid sequence as set out in SEQ ID NO: 2 in the manufacture of a medicament for preventing and/or treating malaria.

17. The use of claim 16, where the medicament is prepared for administration together with an adjuvant chosen from the group consisting of: aluminium hydroxide/MPL, aluminium phosphate/MPL, ASO2, ISA51, IC31, alum, ISA720 and/or MF59.

18. A vaccine comprising the MSP-1 preparation of any of claims 1-4, the modified nucleic acid of claim 6 or 7, the vector of claim 8 or 9, the MSP-1 fragment p83/30 of claim 12 and/or the MSP-1 fragment p38/42 having an amino acid sequence as set out in SEQ ID NO: 2.
